# EUROPEAN PATENT APPLICATION

(11) **EP 4 056 231 A1**
(43) Date of publication of application: **14.09.2022**
(21) Application number: 21161575.2
(22) Date of filing: 09.03.2021
(51) Int. Cl.: A61P 25/28, A61K 45/06, A61K 31/13, A61K 31/138, A61K 31/166, A61K 31/192

(54) **COMPOUNDS FOR USE IN PROGRESSIVE MULTIPLE SCLEROSIS**

(71) Applicant: Fondazione Centro San Raffaele, 20132 Milano (IT); Institut du Cerveau et de la Moelle Epiniere, 75013 Paris (FR); ASSISTANCE PUBLIQUE, HOPITAUX DE PARIS, 75004 Paris (FR); Centre national de la recherche scientifique, 75016 Paris (FR); Institut National de la Santé et de la Recherche Médicale, 75654 Paris Cedex 13 (FR); SORBONNE UNIVERSITE, 75006 Paris (FR); Westfälische Wilhelms-Universität Münster, 48149 Münster (DE); The Regents of the University of California, Oakland, CA 94607-5200 (US); Heinrich Heine University Düsseldorf, 40225 Düsseldorf (DE); Universitätsmedizin der Johannes Gutenberg-Universität Mainz, 55131 Mainz (DE); The Royal Institution for the Advancement of Learning / McGill University, Montréal, Québec H3A 0G4 (CA); Istituto Superiore Di Sanita', 00161 Roma (RM) (IT); Consiglio Nazionale Delle Ricerche, 00185 Roma (IT); IRBM S.P.A., 00071 Pomezia (RM) (IT)
(72) Inventor: The designation of the inventor has not yet been filed
(74) Representative: Capasso, Olga

(57) **Abstract**

The present invention provides compounds able to induce neuroprotection of damaged neurons and boost the remyelination potential of oligodendrocytes. Said compounds have been identified through methods of pharmacological screening on a small molecule library consisting of known pharmacologically active compounds and approved drugs. The screening method is also included in the invention.

## Description

### ABSTRACT

The present invention provides compounds able to induce neuroprotection of damaged neurons and boost the remyelination potential of oligodendrocytes. Said compounds have been identified through methods of pharmacological screening on a small molecule library consisting of known pharmacologically active compounds and approved drugs. The screening method is also included in the invention.

### INTRODUCTION

Multiple sclerosis (MS), an inflammatory autoimmune disease characterized by disruption of myelin and axonal damage, is the major cause of progressive neurological disability in young adults and has a large societal impact.¹⁻⁴ Most MS patients initially have a relapsing-remitting (RRMS) profile, 80% of which enter a phase of continuous accumulation of disability (secondary progressive SPMS). A smaller number of patients experience worsening from onset (primary progressive PPMS). Altogether, around 50% of the 2.3 million of MS patients worldwide live with type of progressive MS (PMS). While RRMS is dominated by the inflammatory response, PMS is the result of different direct and indirect mechanisms damaging myelin, oligodendrocytes and neurons. Indeed, neurodegeneration develops over time as a consequence of the pathological processes of immune-mediated demyelination⁵, but also as a consequence of a direct cytotoxic attack by immune cells^{6,7}, of a dysfunctional neuronal-glia cross talk⁸, and, last, but not least, of the exposure to excitotoxic substances such as glutamate. However, according to recent evidence, intrinsic defects of resident cells within the central nervous system (CNS) cannot be excluded^{9.10}. Examples are oxidative stress¹¹, ion channel dysfunction¹², mitochondrial and energy deficit^{13,14}, all maladaptive changes to white matter axon transection that accelerate degeneration. Furthermore, when the disease progresses, remyelination, a mechanism normally occurring in the adult CNS¹⁵ fails and, although oligodendrocyte precursors (OPCs) are found in the glial scar, they appear locked in an immature status. ^{16,17} Suggested mechanisms for this process include altered semaphorin levels¹⁸, glutamate toxicity¹⁹, or metabolic deficiencies across cell types as consequence of altered neuronal-glia cross-talk.²⁰

While targeting inflammatory pathways has successfully helped to face RRMS, there are no therapies for the PMS, such as drugs promoting remyelination, axonal recovery or neuronal preservation.²¹ Although ∼70,000 RRMS patients are currently participating in clinical trials or observational studies, no more than 1/10 of the patients in trials are progressive, and only very few (∼10) neuroprotective and remyelination trials for PPMS and/or SPMS are ongoing (source: clinicaltrials.gov). Thus, the discovery of agents favoring neuroprotection, remyelination and prevention of cognitive decline is critically needed, and trials enrolling this category of patients cannot be delayed any longer.

We have designed and conducted a comprehensive and well-characterized pharmacological screening to ultimately identify a handful of lead compounds with therapeutic potential for PMS. Our search for consistency across various biological levels and methods of analysis along with data obtained on the mechanism of action and on the pharmacological properties of the compounds, will translate into results that will de-risk any consequent clinical research and investment.

In the last 20 years, only a few breakthrough newly designed drugs have been brought to the market and still some have limited clinical success in RRMS patients and none yet for PMS.² Thus, a rational approach to provide patients with therapies in a relative short period of time is drug repurposing. Repositioned drugs have already been tested preclinically and clinically and their safety, tolerability and pharmacological interactions are mostly known.

### SUMMARY OF THE INVENTION

The present invention provides compounds and compositions comprising said compounds able to:
a) increase oligodendrocyte precursor cell (OPC) differentiation and/or to produce an expanded population of oligodendrocytes and/or to increase remyelination; and b) preserve neuronal viability and morphology.

Therefore, the compounds and compositions of the invention find use in the treatment of neurodegenerative diseases, in particular in neurodegenerative diseases caused by immune demyelination, favoring neuroprotection, remyelination and prevention of cognitive in a subject in need thereof.

It is an object of the invention a compound able to increase oligodendrocyte precursor cell (OPC) differentiation to oligodendrocyte and/or to increase remyelination and/or to preserve neuronal viability and morphology, the compound being selected from:
a) an NK1 receptor inhibitor and/or a Sigma1 receptor inhibitor comprising Casopitant, Aprepitant, Fosaprepitant, Rolapitant, Lanepitant and Orvepitant; and/or
b) an H3R antagonist comprising Bavisant, Pitolisant, GSK189254, PF-03654746, A-331440, JNJ-39220675 and MK-0249; and/or
c) a CGRP antagonist comprising Olgecepant, Telcagepant, BI 44370 TA, MK-3207, Rimegepant, SB-268262 and Ubrogepant; and/or
d) Lemborexant, PD-0325901, Vanoxerine, Indeglitazar, PAC-14028, NS-018, Rupatadine, Efatutazone Hydrochloride, Alprenolol, Danirixin, SU14813, Ezatiostat Hydrochloride, Acumapimod, Tamibarotene, Drinabant, PF-03654746, Ponesimod, Dovitinib, LY-2090314, Taladegib, Progesterone, Roxadustat, Saracatinib, Telatinib, Gandotinib, Equol, BMS-833923, Merestinib, RG7314, Adenine, Hyoscyamine, Solcitinib, Neramexane, Varlitinib, Imidafenacin, Fevipiprant, Itacitinib, Decernotinib, GSK-2636771, SSR180711, Tarenflurbil, Fluocinolone Acetonide, SB-705498, AZD1981, Raxatrigine, Octanoic Acid, Itopride, Nalfurafine hydrochloride, Istradefylline, GS-4997, AZD9056, Vatalanib, and combinations thereof;
for use in the treatment and/or prevention and/or to ameliorate symptoms of neurodegenerative diseases caused by immune-mediated demyelination.

In a preferred embodiment the compound is Casopitant and/or Bavisant and/or Telgacepant and/or Olgecepant and combination thereof.

In a preferred embodiment the compound is selected from Casopitant, Aprepitant, Fosaprepitant, Rolapitant, Lanepitant, Orvepitant and combination thereof.

In a still preferred embodiment the compound is selected from Bavisant, Pitolisant, GSK189254, PF-03654746, A-331440, JNJ-39220675, MK-0249 and combinations thereof.

In a further preferred embodiment the compound is selected from Olgecepant, Telcagepant, BI 44370 TA, MK-3207, Rimegepant, SB-268262, Ubrogepant and combination thereof.

As used herein, the definitions of neurodegenerative disease or neurodegenerative disease caused by immune demyelination or demyelination disease, each refers to: Acute disseminated encephalomyelitis (ADEM); Acute hemorrhagic leukoencephalitis; Acute optic neuritis; Acute transverse myelitis; Adrenoleukodystrophy; Adrenomyeioneuropathy; Alexander Disease; Alzheimer's Disease; aminoacidurias; Amyotrophic Lateral Sclerosis; Anti-MAG peripheral neuropathy; Anti-MOG associated spectrum; Balo concentric sclerosis; Brain injury; CAMFAK Syndrome; Canavan Disease; Carbon monoxide toxicity; Central pontine myelinolysis; Cerebral hypoxia, Cerebral ischemia; Charcot-Marie-Tooth disease; Chronic inflammatory demyelinating polyneuropathy; Chronic relapsing inflammatory optic neuritis (CRION); Chronic traumatic encephalopathy; clinically isolated syndrome (CIS); Congenital Cataract; Copper deficiency associated condition; Delayed Post-Hypoxic Leukoencephalopathy; diffuse cerebral sclerosis of Schilder; diffuse myelinoclastic sclerosis; extrapontine myelinolysis; Gaucher disease; Guillain-Barre syndrome; Hereditary neuropathy; hereditary neuropathy with liability to pressure palsy; HTLV-1 -associated myelopathy; Hurler syndrome; Hypomyelination; hypoxic brain injury; Krabbe Disease; Leber hereditary' optic atrophy and related mitochondrial disorders; leukodystrophic disorders; Marburg multiple sclerosis; Marchiafava-Bignami disease; Metachromatic leukodystrophy; multiple sclerosis, multiple system atrophy; myelinoclastic disorders; myelopathy; nerve injury; neuromyelitis optica; Neuromyelitis optica (NMO); Niemann-Pick disease; optic neuropathy; optic-spinal multiple sclerosis; Osmotic Demyelination Syndrome; Parkinsons; Pelizaeus-Merzbacher Disease; peripheral neuropathy; Phenylketonuria; primary progressive multiple sclerosis (PPMS); progressive inflammatory neuropathy; progressive multifocal leukoencephalopathy; Progressive subcortical ischemic demyelination; progressive-onset multiple sclerosis, relapsing-onset multiple sclerosis, relapsing-remitting multiple sclerosis (RRMS); reperfusion injury; Schilder disease; secondary progressive multiple sclerosis (SPMS); Solitary sclerosis; Spinal Cord Injury; Subacute sclerosing panencephalitis, Tabes dorsalis; Tay-Sachs disease; Traumatic Brain Injury; Tropical spastic paraparesis; Tumefactive multiple sclerosis; or Vitamin B 12 deficiency. In preferred embodiments the demyelination disease is Multiple Sclerosis, Optic-spinal multiple sclerosis, Amyotrophic Lateral Sclerosis, Chronic relapsing inflammatory optic neuritis (CRION), Neuromyelitis optica, or Chronic inflammatory demyelinating polyneuropathy.

In a preferred embodiment, the neurodegenerative disease caused by immune-mediated demyelination is Multiple Sclerosis, Progressive Multiple sclerosis, Optic-spinal multiple sclerosis, Amyotrophic Lateral Sclerosis, Chronic relapsing inflammatory optic neuritis (CRION), Neuromyelitis optica, or Chronic inflammatory demyelinating polyneuropathy.

It is a further object of the invention a pharmaceutical composition comprising at least one compound as defined above or a combination thereof, and a pharmaceutically acceptable carrier for use in the treatment and/or prevention of neurodegenerative diseases caused by immune-mediated demyelination.

Preferably in said pharmaceutical composition said compound is comprised at a concentration of about between 100nM and 100µM; still preferably said compound is Casopitant and/or Bavisant and/or Telcagepant and/or Olgecepant; even more preferably Casopitant and/or Bavisant and/or Telcagepant and/or Olgecepant are comprised at a concentration of about between 100nM and 100µM.

The pharmaceutical composition is for use in the treatment of a neurodegenerative disease caused by immune-mediated demyelination preferably being Multiple Sclerosis, Progressive Multiple sclerosis, Optic-spinal multiple sclerosis, Amyotrophic Lateral Sclerosis, Chronic relapsing inflammatory optic neuritis (CRION), Neuromyelitis optica, or Chronic inflammatory demyelinating polyneuropathy.

According to the invention, the compounds able a) increase oligodendrocyte precursor cell (OPC) differentiation and/or to produce an expanded population of oligodendrocytes and/or to increase remyelination; and b) preserve neuronal viability and morphology, might act through different mechanism of action. As indicated in Table 1, the invention comprises compounds known as: NK1 receptor inhibitors, Sigma1 receptor inhibitor comprising H3R antagonists, CGRP antagonists, H1R and platelet activating factor receptor antagonists, Orexin receptor antagonists, PPAR-gamma agonists, MAPKs inhibitors, dopamine reuptake inhibitors, TRPV1 antagonists, JAK inhibitors, β-AR antagonists, CXCR2 antagonists, RTKs inhibitors, multiple TKs inhibitors, GST inhibitors, retinoid receptor agonists, CB1 receptor antagonists, S1PR3 agonists, GSK3 inhibitors, SMO antagonists, agonists of THRb, Erβ agonists, HGFR inhibitors, vasopressin receptor antagonists, acetylcholine antagonists, NMDA antagonists, EGFR inhibitors, prostaglandin receptor antagonists, PI3k inhibitors, inhibitors of voltage-gated sodium channels, KOR agonists, A2A receptors inhibitors, ASK1 inhibitors. P2X7 receptor antagonists, VEGF receptors antagonists.

**Table 1. Preferred compounds of the invention, relative CAS No. and known mechanism of action.**

| **Compound Name** | **Cas No.** | **Known Mechanism of action** |
|---|---|---|
| Casopitant | 414910-27-3 | NK1 inhibitor |
| Aprepitant | 170729-80-3 | NK1 inhibitor |
| Fosaprepitant | 172673-20-0 | NK1 inhibitor |
| Rolapitant | 552292-08-7 | NK1 inhibitor |
| Lanepitant | 170566-84-4 | NK1 inhibitor |
| Orvepitant | 579475-18-6 | NK1 inhibitor |
| Bavisant | 929622-08-2 | H3R antagonist |
| GSK189254 | 720690-73-3 | H3R antagonist |
| Pitolisant | 362665-56-3 | H3R antagonist |
| A331440 | 1049740-32-0 | H3R antagonist |
| JNJ-39220675 | 959740-39-7 | H3R antagonist |
| MK-0249 | 862309-06-6 | H3R antagonist |
| PF-03654746 | 935840-31-6 | H3R antagonist |
| Telcagepant | 781649-09-0 | CGRP antagonist |
| Olgecepant | 204697-65-4 | CGRP antagonist |
| BI 44370 TA | 866086-05-7 | CGRP antagonist |
| MK-3207 | 957116-20 | CGRP antagonist |
| Rimegepant | 1289023-67-1 | CGRP antagonist |
| SB-268262 | 217438-17-0 | CGRP antagonist |
| Ubrogepant | 1374248-77-7 | CGRP antagonist |
| Rupatadine | 158876-82-5 | H1R and platelet activating factor receptor antagonist |
| Lemborexant | 1369764-02-2 | Orexin receptor type 1,2 antagonist |
| Efatutazone Hydrochloride | 223132-37-4 | PPAR-gamma agonist |
| Indeglitazar | 835619-41-5 | PPAR-alpha, gamma, delta agonist |
| PD-0325901 | 391210-10-9 | MAPK 1,2 inhibitor |
| Acumapimod | 836683-15-9 | p38 MAPK inhibitor |
| Vanoxerine | 67469-69-6 | dopamine reuptake inhibitor |
| PAC-14028 | 1005168-10-4 | TRPV1 antagonist |
| NS-018 | 1239358-86-1 | JAK2 inhibitor |
| Alprenolol | 13707-88-5 | β-AR antagonist |
| Danirixin | 954126-98-8 | CXCR2 antagonist, IL8 beta receptor antagonist |
| SU14813 | 627908-92-3 | RTKs inhibitor |
| Dovitinib | 405169-16-6 | RTKs inhibitor |
| Saracatinib | 379231-04-6 | TKs inhibitor |
| Telatinib | 332012-40-5 | TKs inhibitor |
| Ezatiostat Hydrochloride | 286942-97-0 | Glutathione S-Transferase pi inhibitor |
| Tamibarotene | 94497-51-5 | Retinoic acid alpha, beta receptor agonist |
| Drinabant | 358970-97-5 | CB1 receptor antagonist |
| Ponesimod | 854107-55-4 | S1PR3 agonist |
| LY-2090314 | 603288-22-8 | GSK3 inhibitor |
| Taladegib | 1258861-20-9 | Hedgehog inhibitor, SMO antagonist |
| BMS-833923 | 1059734-66-5 | SMO antagonist |
| Progesterone | 57-83-0 | Progesterone receptor agonist |
| Roxadustat | 808118-40-3 | HIF prolyl hydroxylase inhibitor, |
| Gandotinib | 1229236-86-5 | JAK2 inhibitor |
| Equol | 94105-90-5 | Erβ agonist |
| Merestinib | 1206799-15-6 | RTK antagonist |
| RG7314 | 1228088-30-9 | Vasopressin receptor antagonist |
| Adenine | 73-24-5 | Purine base |
| Hyoscyamine | 101-31-5 | Muscarinic receptor antagonist |
| Solcitinib | 1206163-45-2 | JAK1 inhibitor |
| Itacitinib | 1334298-90-6 | JAK1 inhibitor |
| Decernotinib | 944842-54-0 | JAK3 inhibitor |
| Neramexane | 219810-59-0 | NMDA antagonist |
| Varlitinib | 845272-21-1 | EGFR antagonist |
| Imidafenacin | 893421-54-0 | Muscarinic M1, M3 receptor antagonist |
| F evipiprant | 872365-14-5 | Prostaglandin D2 receptor antagonist |
| AZD1981 | 802904-66-1 | Prostaglandin receptor antagonist |
| GSK-2636771 | 2108806-07-9 | PI3k inhibitor |
| SSR180711 | 446031-79-4 | Partial agonist for the α7 subtype of neural nicotinic acetylcholine receptors |
| Tarenflurbil | 51543-40-9 | NSAID, NF-kb inhibitor, AP 1 transcription factor inhibitor |
| Fluocinolone Acetonide | 67-73-2 | Glucocorticoid receptor agonist |
| SB-705498 | 501951-42-4 | Selective blocker of the TRPV1 ion channel |
| Raxatrigine | 934240-30-9 | Inhibitor of voltage-gated sodium channel protein type 9, alpha subunit blocker |
| Octanoic Acid | 124-07-2 | Ketoglutarate de-hydrogenase complex inhibitor, Pyruvate de-hydrogenase complex inhibitor |
| Itopride | 122898-67-3 | Dopamine D2 antagonist |
| Nalfurafine hydrochloride | 152658-17-8 | KOR agonist |
| Istradefylline | 155270-99-8 | A2A receptors inhibitor |
| GS-4997 | 1448428-04 | MAPK 5 inhibitor, ASK1 inhibitor |
| AZD9056 | 345304-65-6 | P2X7 receptor antagonist |
| Vatalanib | 212141-54-3 | VEGF receptors antagonist |

In a preferred embodiment the compound of the invention is an NK1 inhibitor and/or a Sigma1 receptor inhibitor comprising, preferably selected from Casopitant, Aprepitant, Fosaprepitant, Rolapitant, Lanepitant, Orvepitant and/or combinations thereof.

In a still preferred embodiment the compound of the invention is a CGRP antagonist, preferably selected from Olgecepant, Telcagepant, BI 44370 TA, MK-3207, Rimegepant, SB-268262, Ubrogepant and/or combinations thereof.

In a further preferred embodiment of the invention, the compound of the invention is a H3R antagonist, preferably selected from Bavisant, Pitolisant, GSK-189254, PF-03654746, A-331440, JNJ-39220675, MK-0249 and/or combinations thereof.

It is a further object of the invention a method for identifying a compound able to increase oligodendrocyte precursor cell (OPC) differentiation and/or to produce an expanded population of oligodendrocytes, wherein said method comprises:
- a toxicity assay on neonatal mouse oligodendrocyte progenitor cells wherein test compounds are screened for their ability to reduce [3-(4,5-dimethylthiazol-2-yl)-2,5-diphenyltetrazolium bromide (MTT)] through the following steps; and/or
- a toxicity assays on rat oligodendrocyte progenitor double fluorescence CG4 line; and/or
- a differentiation assay on rat oligodendrocyte progenitor CG4 line It is a further object of the invention a method for identifying a compound able to preserve neuronal viability and morphology in cell culture, wherein said method comprises:
- a toxicity assay on primary mouse cortical neurons wherein test compounds are screened in a Cell Counting Kit-8 (CCK-8) assay using WST-8 (2-(2-methoxy-4-nitrophenyl)-3-(4-nitrophenyl)-5-(2,4-disulfophenyl)-2H-tetrazolium, monosodium salt); and/or
- a neuroprotective assay on primary mouse cortical and striatal neurons wherein compounds are screened for their ability to preserve neuronal viability and morphology (neurite length and network integrity/branching) against NMDA-induced excitotoxicity; and/or
- a toxicity assay on iPSC-derived glutamatergic neurons wherein test compounds are screened in a Cell Counting Kit-8 (CCK-8) assay using WST-8 (2-(2-methoxy-4-nitrophenyl)-3-(4-nitrophenyl)-5-(2,4-disulfophenyl)-2H-tetrazolium, monosodium salt); and/or
- a neuroprotective assay on iPSC-derived glutamatergic neurons wherein compounds are screened for their ability to preserve neuronal viability against ROS (tBuOOH)-induced toxicity.

It is a further object of the invention a method for identifying a compound able to increase neuronal differentiation and/or to produce an expanded population of neurons in cell culture, wherein said method comprises a differentiation assay on human fetal NPCs towards TUJ1+ neuronal precursors wherein:
- test compounds at 1µM concentration are co-incubated with hufNPCs for 7 days;
- 0.02% vol/vol DMSO and basal medium are used as negative controls;
- heparin is used as a positive control;
- at the end of incubation cells are fixed and analyzed via immunocytochemistry.

It is a further object of the invention a compound identified by anyone of the above methods for use in the treatment and/or prevention and/or to ameliorate symptoms of neurodegenerative diseases caused by immune-mediated demyelination; or pharmaceutical composition comprising at least one compound identified by said methods and a pharmaceutically acceptable carrier for use in the treatment and/or prevention and/or to ameliorate symptoms of neurodegenerative diseases caused by immune-mediated demyelination; preferably said neurodegenerative diseases are selected from Multiple Sclerosis, Progressive Multiple sclerosis, Optic-spinal multiple sclerosis, Amyotrophic Lateral Sclerosis, Chronic relapsing inflammatory optic neuritis (CRION), Neuromyelitis optica, or Chronic inflammatory demyelinating polyneuropathy.

It is a further object of the invention the use of a compound as described above in a method in vitro to increase oligodendrocyte precursor cell (OPC) differentiation to oligodendrocyte and/or to increase remyelination and/or to preserve neuronal viability and morphology in a cell culture.

Compounds suitable as embodiments of the invention include one or more compounds selected form the group comprising:
Casopitant, Bavisant, Telcagepant, Lemborexant, GSK189254, PD-0325901, Vanoxerine, Indeglitazar, PAC-14028, NS-018, Rupatadine, Efatutazone Hydrochloride, Alprenolol, Danirixin, SU14813, Ezatiostat Hydrochloride, Acumapimod, Tamibarotene, Drinabant, PF-03654746, Ponesimod, Dovitinib, LY-2090314, Taladegib, Progesterone, Roxadustat, Saracatinib, Telatinib, Gandotinib, Equol, Olgecepant, BMS-833923, Merestinib, AZD9056, Vatalanib. RG7314, Adenine, Hyoscyamine, Solcitinib, Neramexane, Varlitinib, Imidafenacin, Fevipiprant, Itacitinib, Decernotinib, GSK-2636771, SSR180711, Tarenflurbil, Fluocinolone Acetonide, SB-705498, AZD1981, Raxatrigine, Octanoic Acid, Itopride, Amg-319, Nalfurafine Hydrochloride, OC000459, Pamapimod, L-Serine, CP-724714, Zibotentan, Istradefylline, Vatalanib, Trimebutine, Alprenolol, Ellagic Acid, Etazolate, Benztropine Mesylate, Encenicline, Tenofovir, SDX-101, APD334, GS-4997, Doramapimod, Vidupiprant, Seliciclib, Velneperit, Netoglitazone, Prinaberel, Flindokalner, Elinogrel, Raseglurant, L-Phenylalanine, Talmapimod, Bifeprunox, Mitiglinide, AZD-7624, Ibipinabant, Tideglusib, Basimglurant, Indiplon, Defactinib, Capmatinib, Orteronel, Oliceridine, Ramatroban, VX-702, AMG-208, Emixustat Hydrochloride, K-877 Pemafibrate, Reminertant, Derenofylline, Basimglurant, Losmapimod, Marimastat, Rupatadine, Quizartinib.

As used herein an H3R antagonist is a H3 receptor antagonist, that is a classification of drugs used to block the action of histamine at the H3 receptor; a CGRP antagonist is a compound acting as antagonist of the calcitonin gene-related peptide receptor (CGRPR); a NK1 receptor inhibitor or NK1 inhibitor is a compound interacting with the Neurokinin 1 (NK1) receptor.

As used herein, a compound able to increase oligodendrocyte precursor cell (OPC) differentiation and/or to increase remyelination is a compound inducing, promoting, or enhancing the differentiation and/or proliferation of Oligodendrocyte Progenitor Cells (OPCs) into mature oligodendrocytes to create new myelin sheaths on demyelinated axons in the central nervous system (CNS) and peripheral nervous system (PNS). When OPCs are treated with a compound of the invention, whether the population is *in vivo* or *in vitro,* the treated OPCs have the capacity to proliferate and/or differentiate and, more specifically, differentiate into oligodendrocytes. In some instances, a compound induces and maintains the OPCs to produce daughter OPCs that can divide for many generations and maintain the ability to have a high proportion of the resulting cells differentiate into oligodendrocytes. In certain embodiments, the proliferating OPCs express progenitor cell marker(s) selected from one or more of NG2, PDGFR-alpha, Sox10, NKx2.2. In some embodiments, the compounds may be used to maintain, or even transiently increase self-renewal of a pre-existing progenitor cell population prior to significant myelin sheath formation. Morphological analyses with immunolabeling may be used to confirm expansion of the OPCs and upregulation of markers of mature oligodendrocytes, including MBP, CNPase and SOX10 amongst the cell population. As used herein, a compound able to preserve neuronal viability and morphology is a compound able to induce and/or increase remyelination of a neuronal axon. Remyelination is the process of propagating, proliferating, differentiating, and/or migration of oligodendrocyte precursor cells to form oligodendrocytes and thereby create new myelin sheaths on demyelinated axons in the peripheral or central nervous system. Typically, evidence that remyelination has taken place on an axon includes the creation of a relatively thin myelin sheath which can be quantified by the myelin area and total area of myelinated axons. Alternatively, that evidence of remyelination has taken place on an axon included determining the percentage of axons that are myelinated compared to control.

### BRIEF DESCRIPTION OF THE DRAWINGS

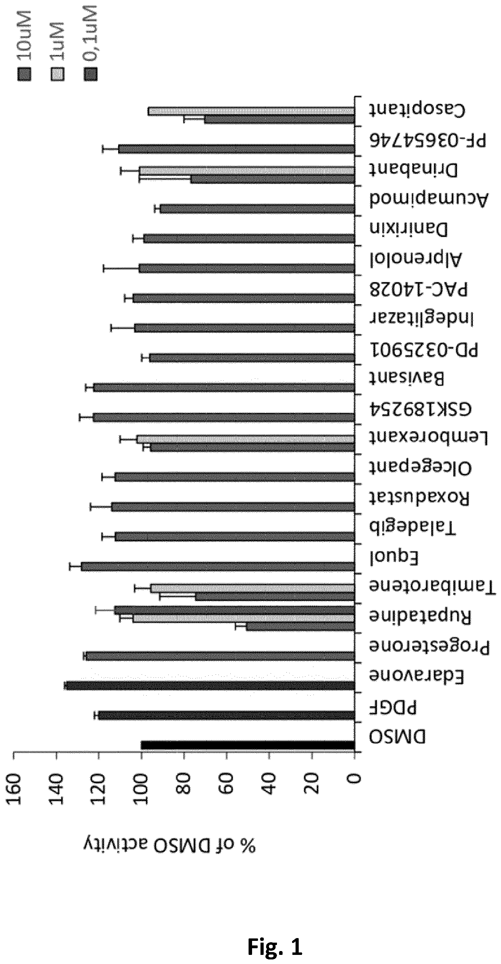

**Figure 1****. Drugs able to stimulate OPC metabolic activity.** The plot shows actives among the 32 Front Runners in MTT test. Cells were treated with compounds (10, 1 and 0.1 µM) or DMSO (0.001% vehicle) for 48 hours. Drug effects were quantified as the % of DMSO activity (absorbance of drug/absorbance of vehicle). Edaravone (10 µM) and PDGF (20 ng/ml) were used as positive controls. Only the compounds with an inhibitory effect on OPC metabolism at 10 µM were tested at lower concentrations. Values are expressed as mean ± SEM of 3-5 different experiments run in triplicates.

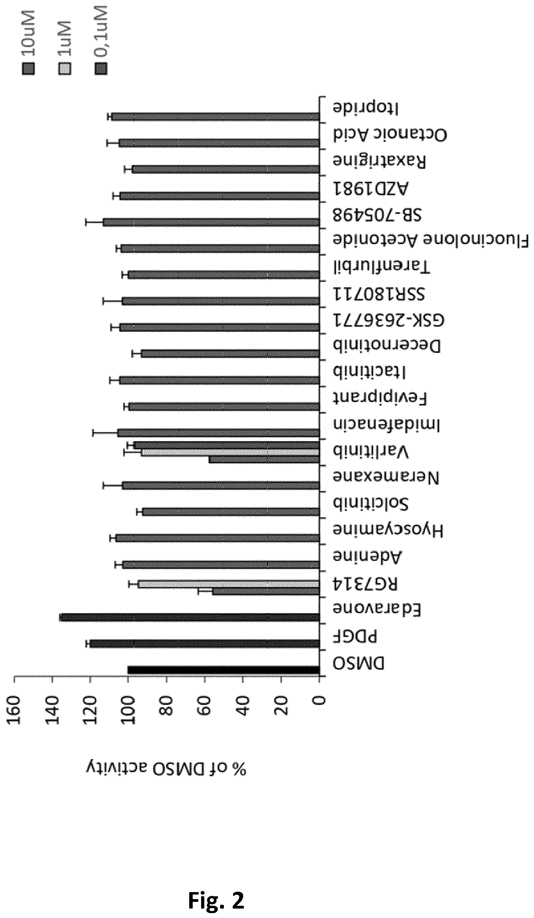

**Figure 2****. Drugs able to stimulate OPC metabolic activity.** The plot shows drugs among the 24 hits selected from the Short List with some stimulatory activity in the MTT test. Cells were treated with compounds (10, 1 and 0.1 µM) or DMSO (0.001% vehicle) for 48 hours. Drug effects were quantified as the % of DMSO activity (absorbance of drug/absorbance of vehicle). Edaravone (10 µM) and PDGF (20 ng/ml) wereused as positive controls. Only the compounds with an inhibitory effect on OPC metabolism at 10 µM were tested at lower concentrations. Values are expressed as mean ± SEM of 3-5 different experiments run in triplicates.

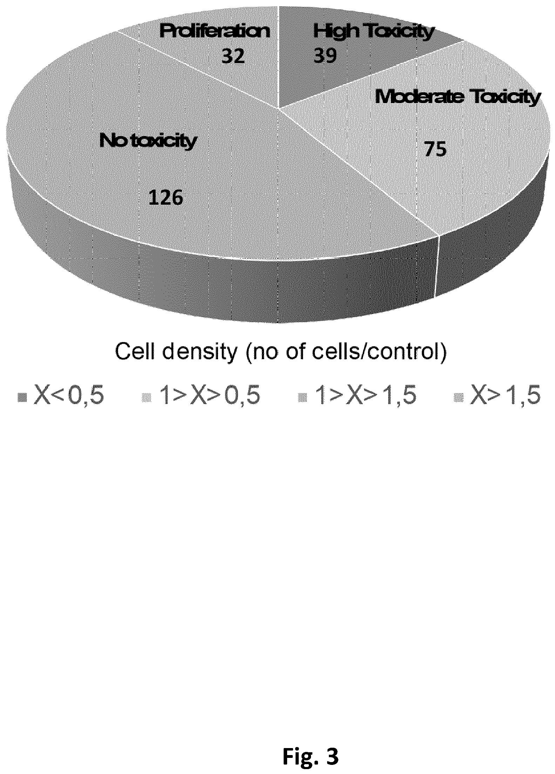

**Figure 3****. Classification of 272 compounds toxicity on CG4 line.** The full list of compounds (272) at 1 µM, using a double fluorescence CG4 cell line (rat OPC line), 9 cis-retinoic acid (1µM) served as a positive control. Toxicity was estimated, based on the compounds' effect on cell density: 32/272 (11.7%) compounds promoted OPC proliferation (fold change > 1.5), 126/272 (46%) resulted in no toxicity (1< fold change < 1.5), 75/272 (27.5%) were moderately toxic (0.5 < fold change < 1.0), while 39/272 (14.3%) exhibited high toxicity (fold change < 0.5).

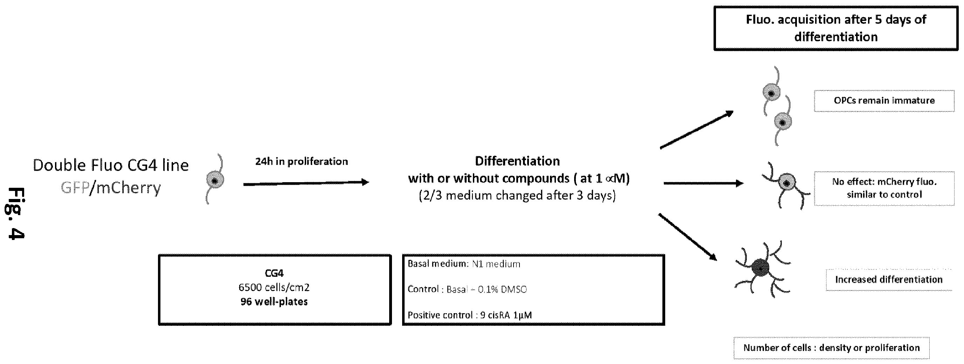

**Figure 4****. Schematic representation of HCS validation of 274 compounds identified through in silico screen using CG4 line**

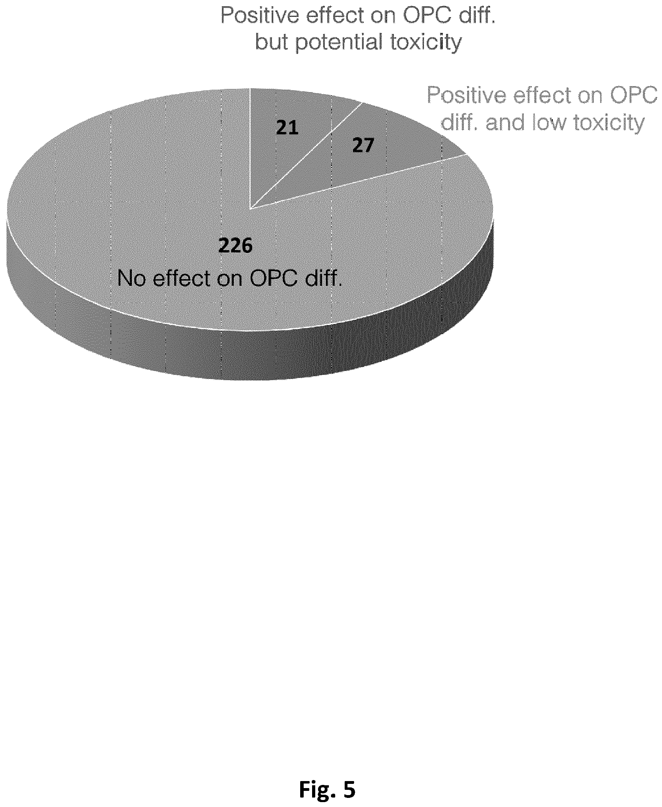

**Figure 5****. Classification of 274 compounds differentiation potential on CG4 line.** 274 tested compounds were also classified according to their ability to induce OPC differentiation: 226/274 (82.5%) compounds did not have effect on OPC differentiation, 21/274 (7.7%) compounds had positive effect but potential toxicity, while 27/274 (9.8%) compounds promoted OPC differentiation and exhibited a low toxicity (mCherry + OLs plus compounds/mCherry + OLs plus N1 medium).

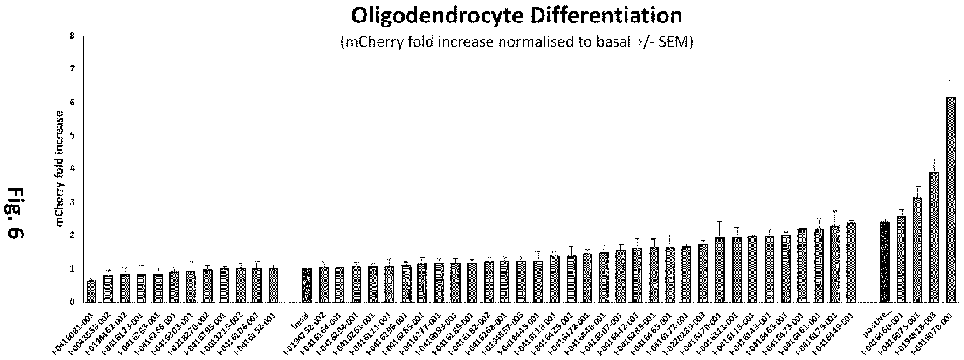

**Figure 6****. Secondary screen of 49 selected compounds on CG4 line differentiation.** 49 hit compounds were identified among 160 non-toxic compounds that increase differentiation either of CG4 cells or primary OPCs. Among hit compounds (promoting differentiation significantly higher than 9cis-RA positive control: 1-0416460-001 (Neramexane), 1-0416075-001 (Quizartinib), 1-0194818-003 (Dovitinib), 1-0416078-001 (LY-2090314). 1-0416081-001 (TALADEGIB), 1-0043558-002 (VANOXERINE), 1-0194462-002 (RUPATADINE), 1-0416123-001 (GANDOTINIB), 1-0416283-001 (PAC-14028), 1-0416266-001 (BAVISANT), I-0416303-001 (ACUMAPIMOD), I-0218270-002 (SARACATINIB), 1-0416295-001 (ALPRENOLOL), 1-0013215-002 (PROGESTERONE), 1-0416106-001 (ROXADUSTAT), 1-0416152-001 (PONESIMOD), I-0194758-002 (EQUOL), I-0416164-001 (BMS-833923), 1-0416294-001 (EFATUTAZONE HYDROCHLORIDE), 1-0416261-001 (LEMBOREXANT), 1-0416111-001 (TELATINIB), I-0416296-001 (DANIRIXIN), I-0416265-001 (GSK189254), I-0416277-001 (INDEGLITAZAR), I-0416093-001(PLX-3397), I-0416189-001 (OLCEGEPANT), I-0416182-002 (EZATIOSTAT HYDROCHLORIDE), I-0416268-001 (PD-0325901), I-0194657-003 (TAMIBAROTENE), I-0416445-001 (CASOPITANT), I-0416118-001 (MGCD-265), I-0416429-001 (DRINABANT), I-0416472-001 (ASIMADOLINE), I-0416448-001 (ETAZOLATE), I-0416307-001 (STANOLONE), I-0416442-001 (PF-03654746), I-0416285-001 (NS-018), I-0416465-001 (CHOLINE ALFOSCERATE), I-0416172-001 (OC000459), I-0220289-003 (ELLAGIC ACID), I-0416470-001(SETIPIPRANT), I-0416311-001 (SU14813), I-0416113-001(REBASTINIB), I-0416143-001(DABIGATRAN), I-0416463-001 (DARUSENTAN), I-0416473-001 (MERESTINIB), I-0416461-001 (BENZATROPINE), I-0416179-001 (AMG-337), I-0416446-001 (LUCITANIB).

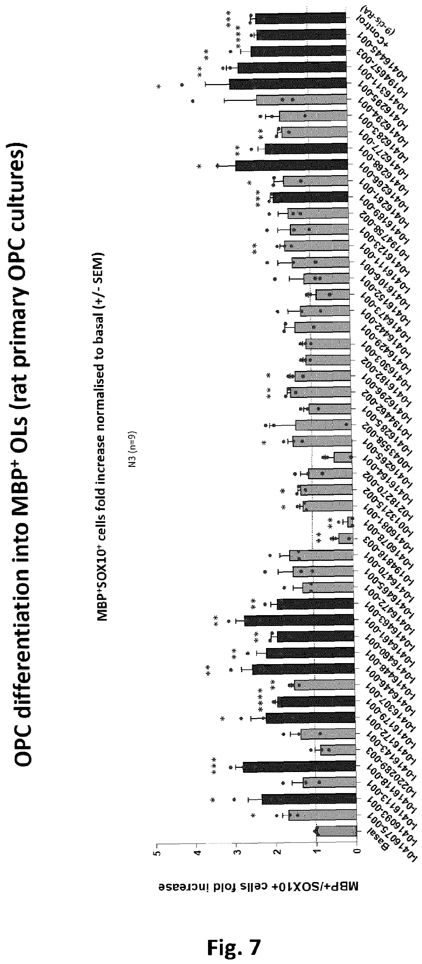

**Figure 7****. Re-validation of 49 selected compounds on primary rat OPC line differentiation.** The secondary validation screen of 49 compounds performed on rat primary OPC cultures selected 17 best lead compounds with a pro-differentiation activity (MBP+/SOX10+ OLs plus compounds/MBP+SOX10+ plus basal medium fold increase) induced a strong differentiation into MBP+ OLs (fold increase >1.6). Image acquisition performed after 4 days of differentiation with compounds tested at 1µM in N=3 independent experiments. In each experiment, compounds were tested in triplicates. Student t-test (* P<0.05; **P<0.01; ***P<0.001; **** P>0.0001). Among hit compounds (effect on MBP+ differentiation > 1.6 fold change): I-0416111-001 (TELATINIB), I-0416261-001 (LEMBOREXANT), I-0416277-001 (INDEGLITAZAR), I-0416283-001 (PAC-14028), I-0416075-001 (QUIZARTINIB), I-0416189-001 (OLCEGEPANT), I-0416460-001 (NERAMEXANE), 1-0416463-001 (DARUSENTAN), I-0416179-001 (AMG-337), I-0416268-001 (PD-0325901), I-0416448-001 (ETAZOLATE), I-0416172-001 (TIMAPIPRANT), I-0416445-001 (CASOPITANT), I-0416093-001 (PLX-3397), I-0194657-003 (TAMIBAROTENE), I-0416446-001 (LUCITANIB), I-0416311-001 (SU14813), I-0416461-001 (Benzatropine), I-0416266-001 (BAVISANT), I-0416118-001 (MGCD-265), I-0416295-001 (ALPRENOLOL).

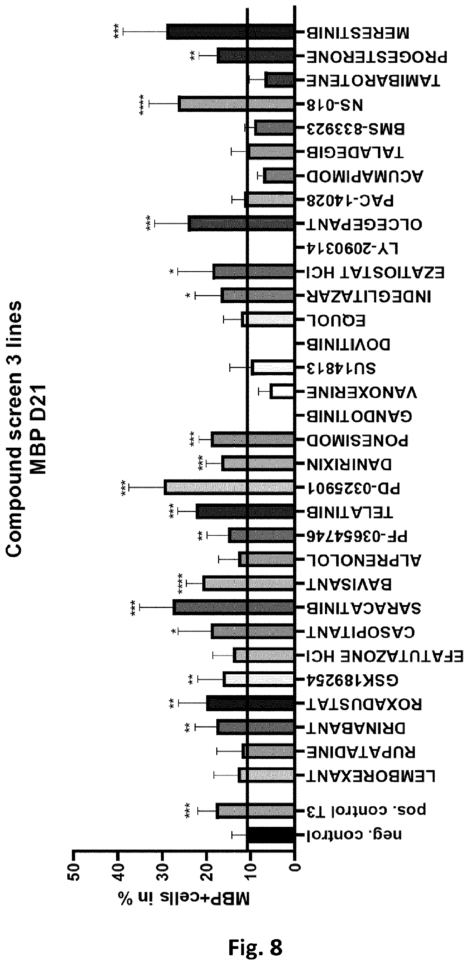

**Figure 8****. Evaluation of differentiation potential of 32 "Front Runners" compounds into MBP+ iPS-derived oligodendrocytes.** The following compounds were able to promote significantly (p<0.05) the differentiation into MBP+ oligodendrocytes: Drinibant, Roxadustat, GSK189254, Casopitant, Saracatinib, Bavisant, PF-03654746, Telatinib, PD-0325901, Danirixin, Ponesimod, Indeglitazar, Ezatiostat hydrochloride, Olcegepant, NS-018, Progesterone and Merestinib.

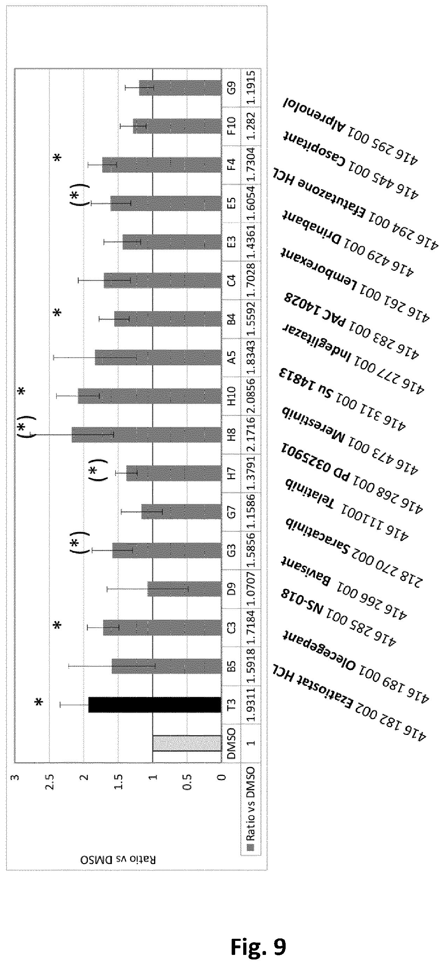

**Figure 9****. Effect of 16 Front Runners on human fetal OPC differentiation into oligodendrocyte (OL).** 16 compounds were evaluated on human fetal neural precursors (hufNPCs) differentiation and identified 4 that significantly promoted fNPCS differentiation (fold change>1.5, p<0.05). These compounds were Olcegepant, Merestinib, Indeglitazar, Efatutazone hydrochloride. In addition, four compounds namely Bavisant, Telatinib, PD0325901 and Drinabant promoted fNPCS differentiation with moderate significance (fold change>1.3, 0.05<p<0.07). Data represent fold increase of OL differentiation based on the % of GalC+ OLs forming sheets vs CC1+ cells with compounds/DMSO at 7 days of differentiation. Compounds were tested in triplicates in 3 independent experiments

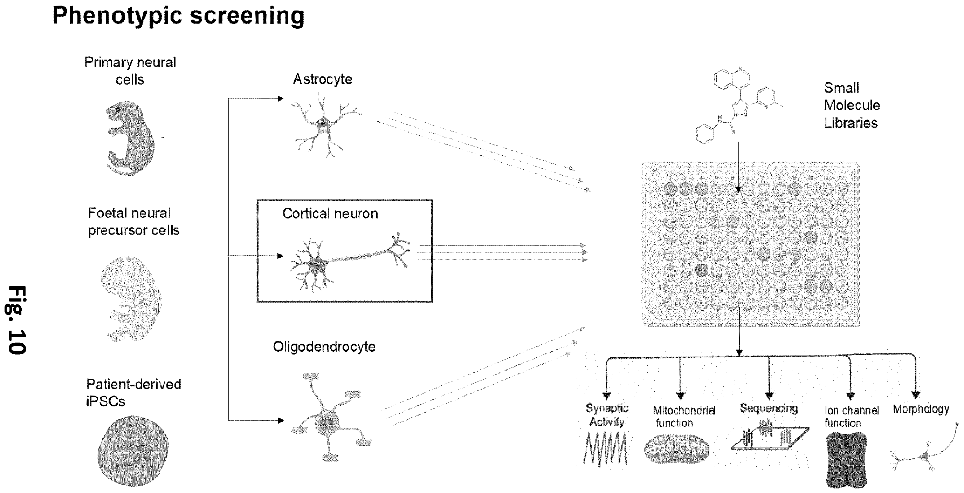

**Figure 10****. Neuronal based assays.** Schematic representation of neuronal-based assay screening strategy.

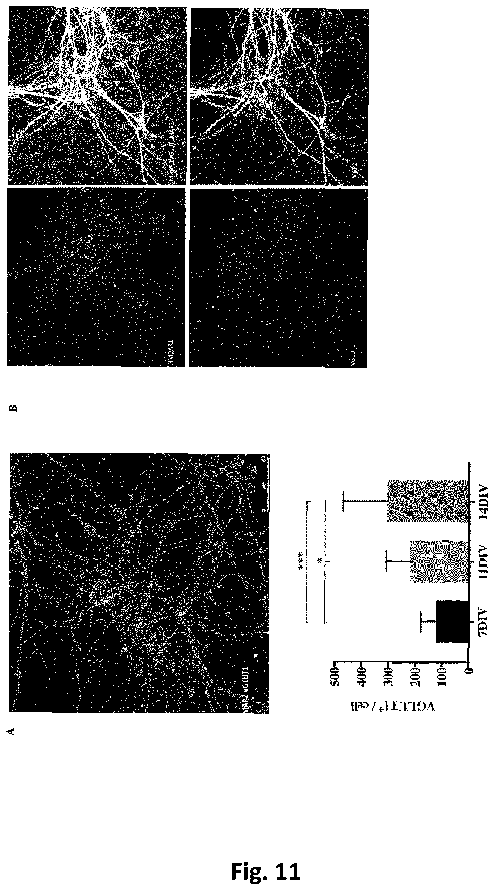

**Figure 11****. Characterization of primary culture of murine cortical neurons.** Representative expression of neural (MAP2) and pre-synaptic (vGLUT1) markers of mouse cortical neurons at 7DIV evaluated by immunolabeling. (B) Representative expression of neural (MAP2), pre-synaptic (vGLUT1) and NMDAR1 markers of mouse cortical neurons at 14DIV evaluated by immunolabeling (C) Quantification of the expression of vGLUT1 normalized by total number of cells. Mean values reported with standard deviation (SD). Stat. test performed with one-way ANOVA, * p < 0.05; *** p < 0.001.

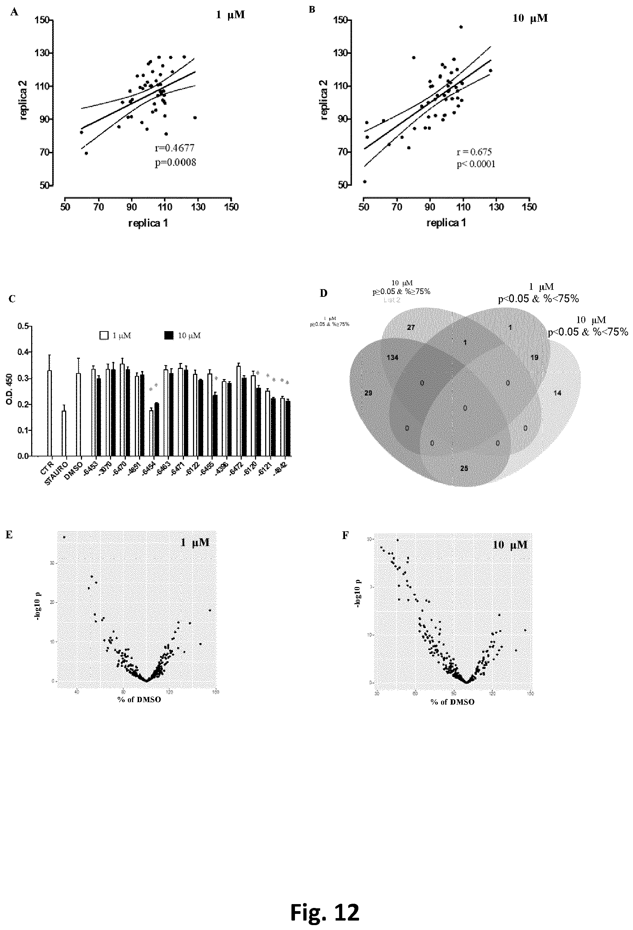

**Figure 12****. Results of the cytotoxicity assay of 274 in silico prioritized compounds evaluated on primary murine cortical neurons.** (A-B) Correlation plot of 47 molecules tested in duplicate to evaluate the reproducibility of toxicity at 1 µM (A) and 10 µM (B). The correlations are visualized with linear regression lines with confidence intervals and Spearman correlation coefficient, significant at the level of p<0.05. (C) Example of cytotoxicity plot measured by CCK8 assay of 14/274 tested compounds. The asterisk indicates the compounds whose viability percentage is significantly lower than DMSO treatment (p≤0.05). Mean values reported with standard deviation (SD). The optical density (O.D. 450) measured at 450 nm is reported on the left axis. (D) Venn's diagram of 274 compounds tested at the two different doses grouped by 75% threshold of viability and p<0.05 stat. significance. (E-F) Volcano plots of 274 compounds tested at 1µM and 10 µM. Y-axis expressed as -log10 p value; x axis is expressed as % to DMSO.

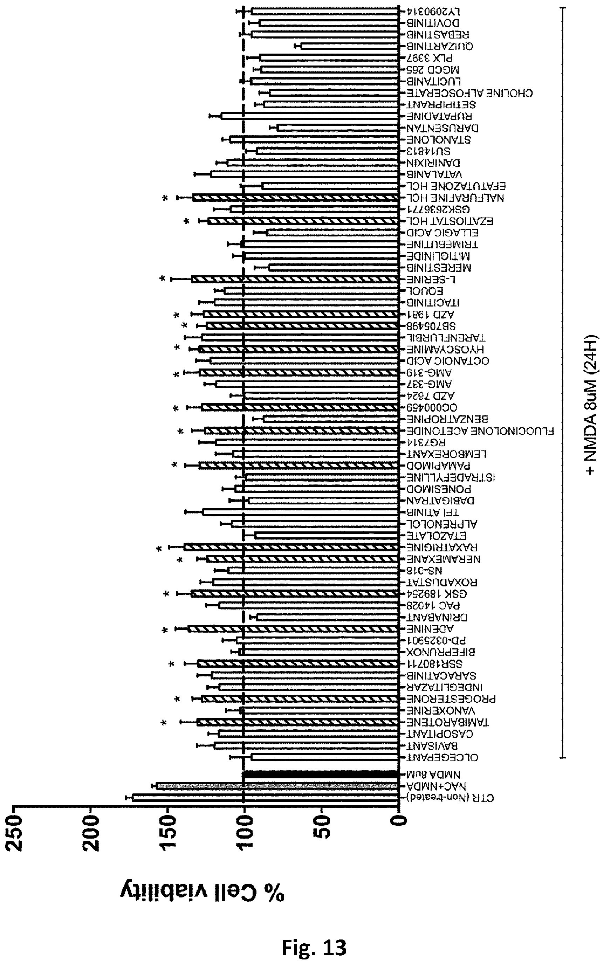

**Figure 13****. Screening of selected non-toxic compounds in NMDA-mediated and neuroprotective assay setting.** Neuroprotective effect of selected compounds on viability/metabolic activity of murine cortical neurons at 14 DIV was evaluated in NMDA-mediated cytotoxic assay by CCK8 kit with 3h compound pre-treatment. The normalized viability/metabolic activity is expressed with respect to the NMDA-treated control. Each bar reports a mean value ± SEM obtained from n > 3 experiments run in triplicates. Stat. test performed with Kruskal-Wallis test with post-hoc two-stage linear step-up procedure of Benjamini, Krieger and Yekutieli multiple comparison correction, *p < 0.05.

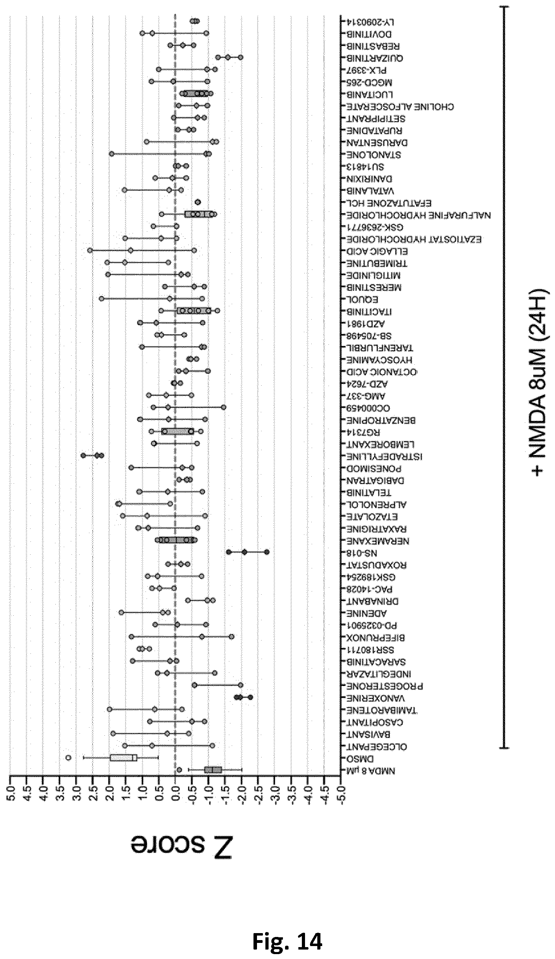

**Figure 14****. Screening of selected non-toxic compounds in NMDA-mediated and neuroprotective assay setting.** Neuroprotective effect of selected compounds on viability/metabolic activity of murine cortical neurons at 14 DIV was evaluated in NMDA-mediated cytotoxic assay by CCK8 kit with 24h compound pre-treatment. The normalized viability/metabolic activity is expressed with Z score. Each boxplot reports a triplicate readout from a single or duplicate experiment.

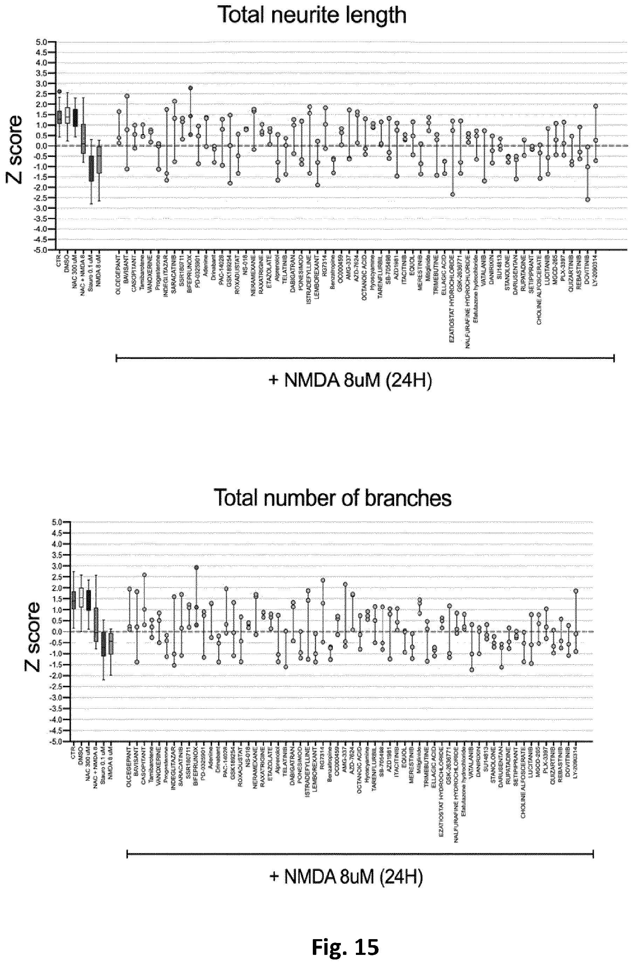

**Figure 15****. Morphological integrity evaluation of mouse cortical neurons (14DIV) upon NMDA (8 µM) cytotoxic exposure.** 160 selected compounds were added 3h prior to NMDA insult in neuroprotective regimen. Total neurite length (Top panel) and total number of branches (bottom panel) expressed as Z-scores were evaluated from 15 images/well, each compound/control was tested in triplicates.

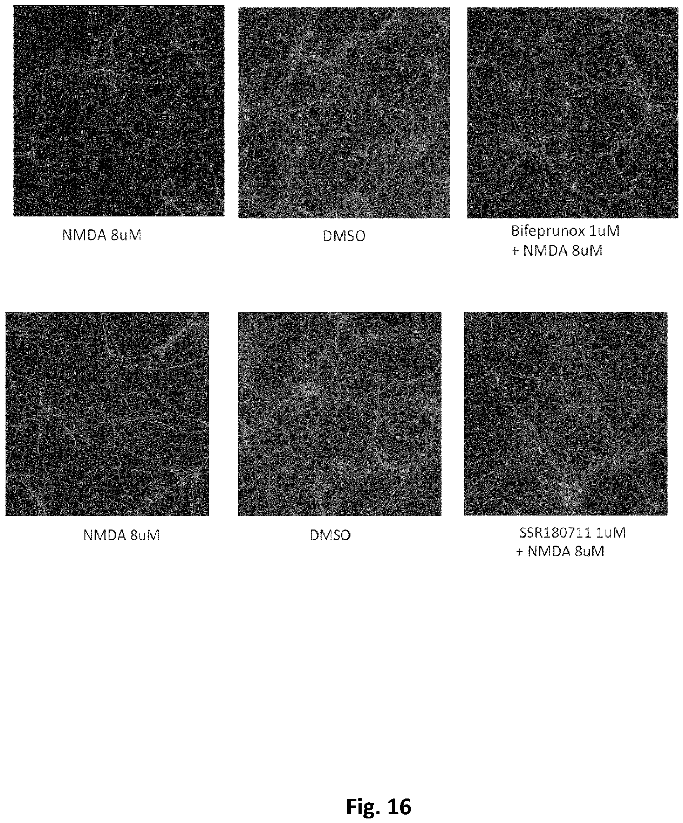

**Figure 16****. The microscopic images of the two best-performing compounds BIFEPRUNOX and SSR180711 in morphological integrity evaluation, potentially NMDA-neuroprotective.** Representative images of mouse cortical neurons (DIV14) immunolabelled for MAP2, where NMDA 8 µM served as a stressor and DMSO (0.02% vol/vol) as a positive control.

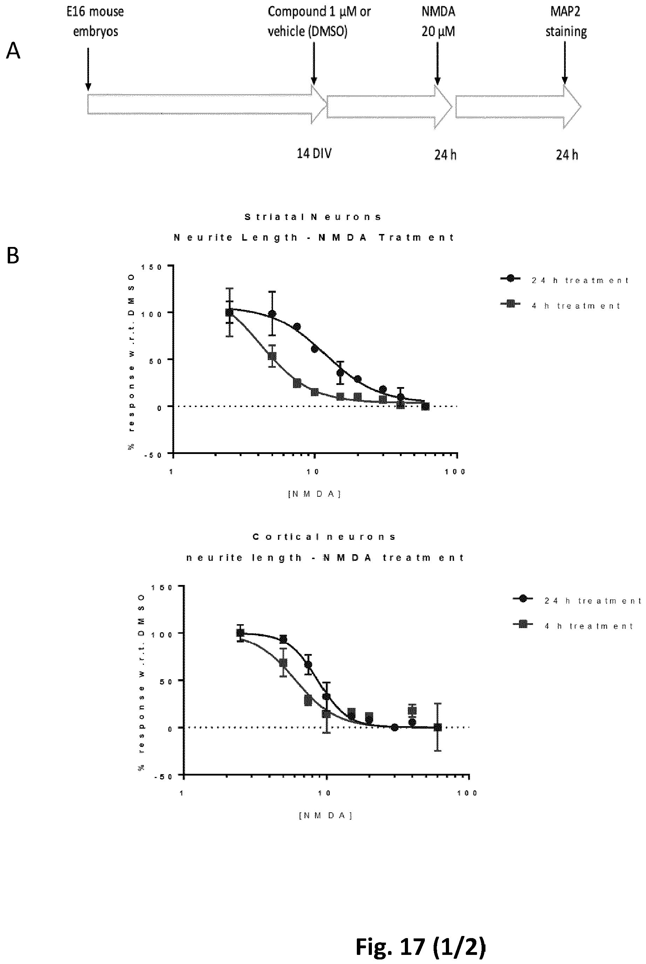

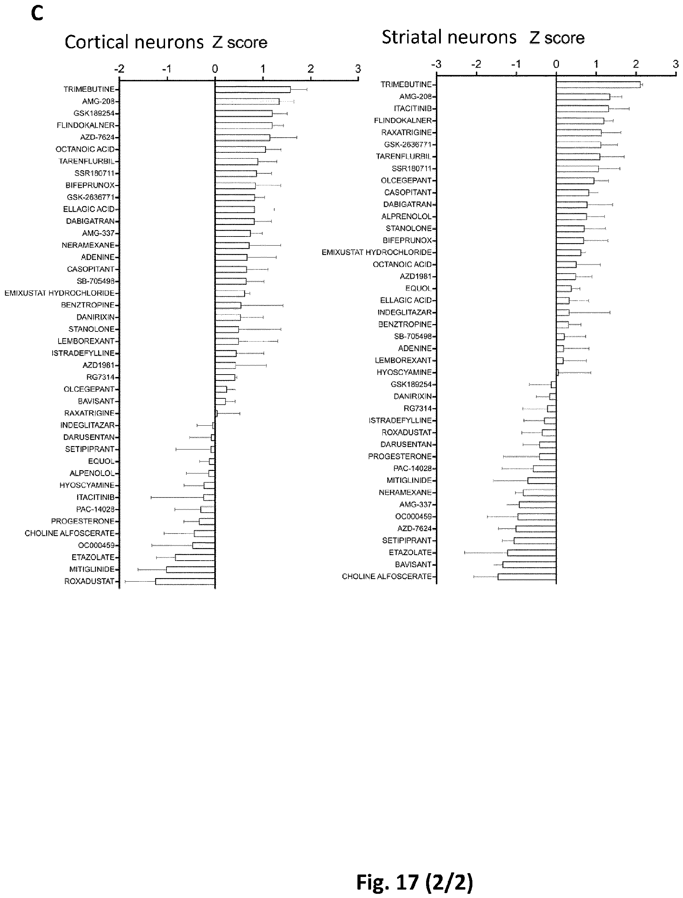

**Figure 17****. Morphological neuronal integrity evaluation.** The schematic representation (A) of the assay evaluating morphological integrity of mouse cortical and striatal neurons (14DIV) upon cytotoxic exposure of several doses of NMDA (4h vs. 24h treatment) (B). Evaluation of neurite length of cortical and striatal in NMDA-mediated assay (C): 42 compounds were screened to evaluate the morphological integrity of cortical and striatal neurons upon chronic (24h) NMDA exposure at concentration of 20µM with 1µM compounds pre-treatment (24h) in preventive regimen. The total neurite length of cortical and striatal neurons is expressed as Z-scores and SDs. Each compound was tested in quadruplicate

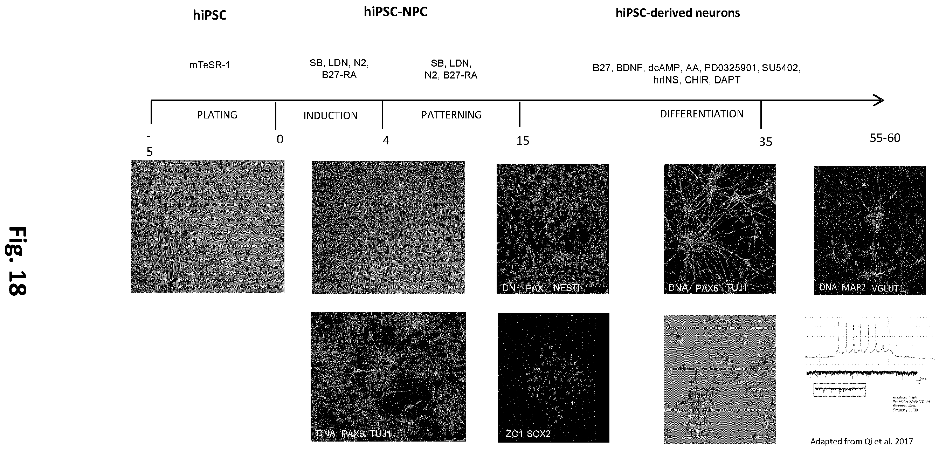

**Figure 18****. Scheme for the generation of glutamatergic neurons from iPSC.** hiPSCs were obtained by reprogramming from skin fibroblasts with a small-molecule approach. iPSCs were further differentiated into neural precursor cells (NPC), and further into glutamatergic neurons

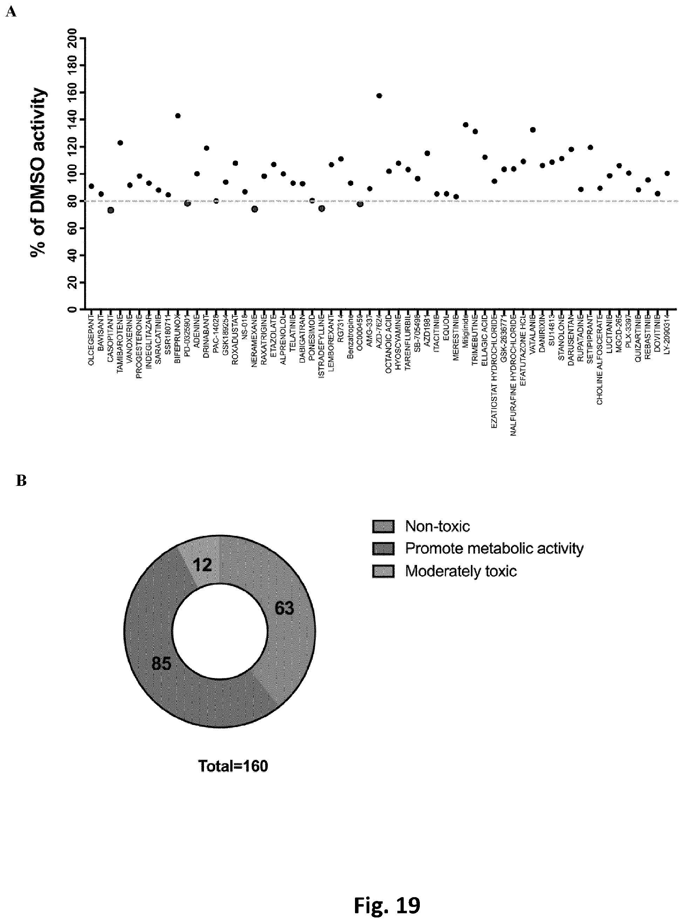

**Figure 19****. Evaluation of cytotoxicity of selected compounds on iPSC-derived neurons (DIV45).** Cytotoxicity of 61 compounds was evaluated on iPSC-derived neurons (DIV45) by CCK8 kit assay. (A) Cell viability/metabolic activity is expressed in percentage relative to DMSO activity. The dotted line marks a threshold of 80% viability/metabolic activity, where each dot reports a mean value of a triplicate readout. (B) 148/160 compounds were considered non-toxic (>80% of metabolic activity relative to DMSO) while 12/160 compounds were moderately toxic.

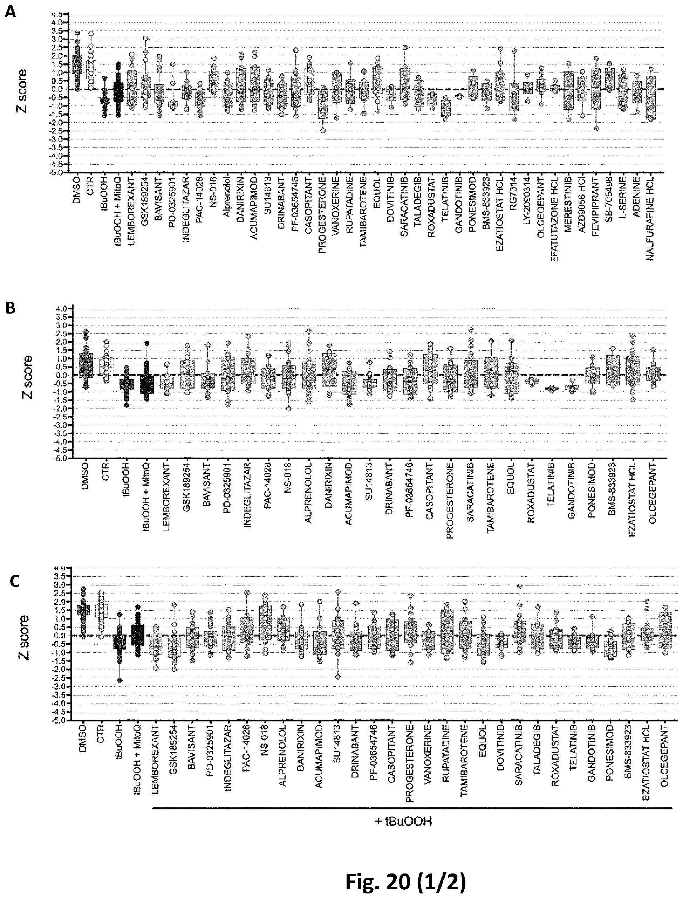

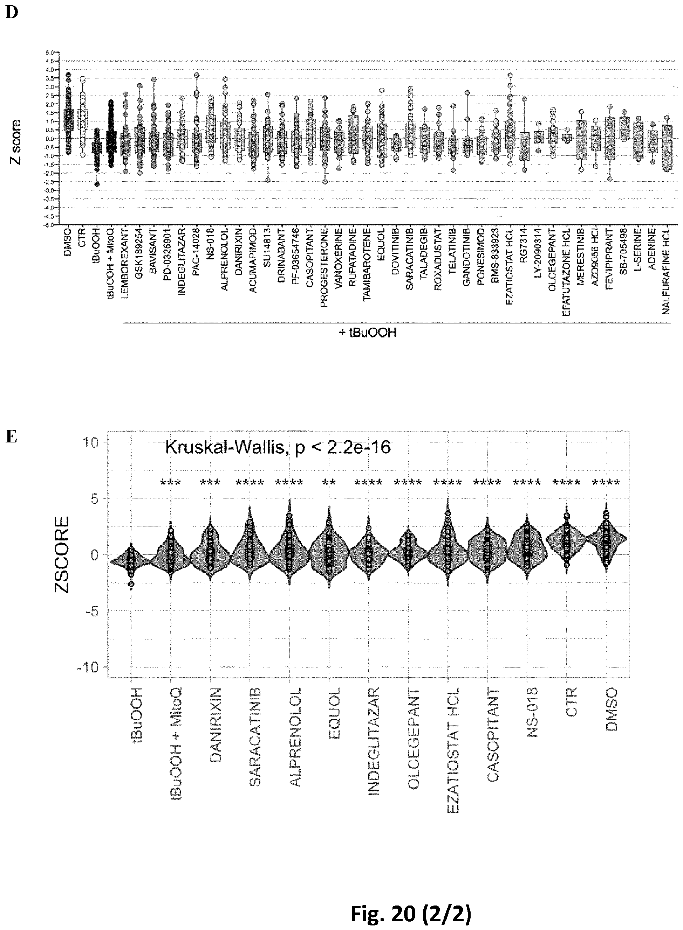

**Figure 20****. Evaluation of neuroprotective potential of 39 selected compounds on iPSC-derived neurons derived from three healthy control lines in ROS-mediated assay.** Compounds were tested in three control lines CTR4 (A), CTR7 (B), CTR8 (C) in three independent experiments, each compound was run in sextuplicate. Each boxplot represents the median value, and each replica is shown as a single dot. Each 96 well plate included controls: 0.02% DMSO, CTR (non-treated), tBuOOH (positive control (stressor) and combination of tBuOOH + MitoQ served as a positive neuroprotective control. Summary of nine independent experiments performed on three control lines is plotted in D. Violin plots of the nine compounds stat. significant at p<0.05 (Kruskal-Wallis test with post-hoc Dunn's multiple comparisons correction) compared to tBuOOH, potentially neuroprotective from tBuOOH-mediated toxicity.

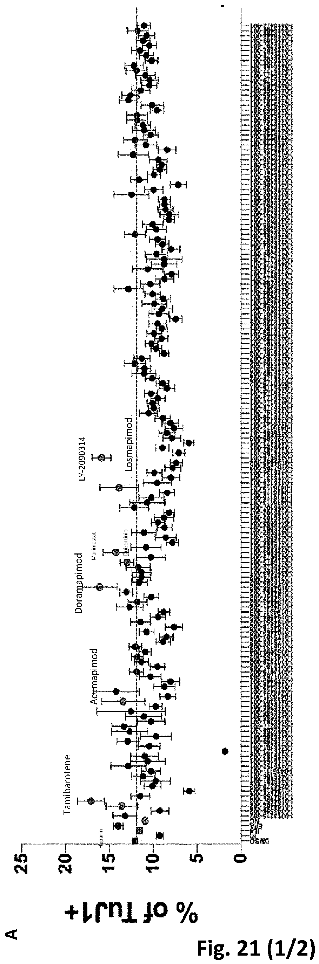

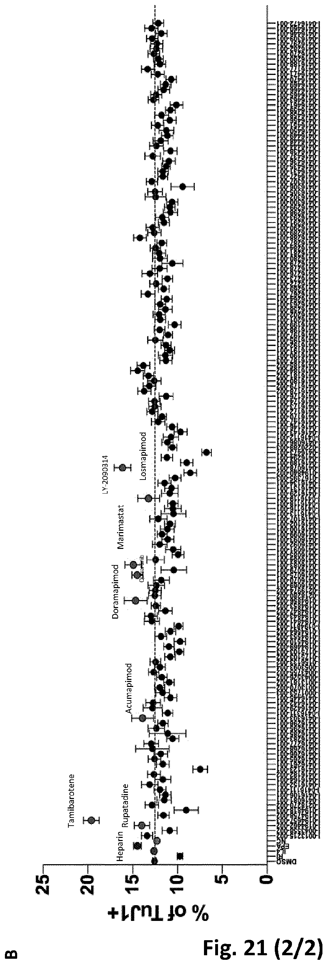

**Figure 21****. Evaluation of pro-differentiating potential of 160 selected compounds on hufNPCs.** Compounds were tested twice (replica 1 is shown in A, replica 2 is shown in B), each compound run in triplicates. Percentage of TUJ1+ cells/DAPI evaluated 15 images/well was compared to DMSO, while heparin served as positive control promoting neuronal differentiation. Each dot represents mean value obtained from 45 images ± SEM. Each 96 well plate included controls: 0.02% DMSO, basal (non-treated), RI (Rock inhibitor), Heparin, IL-4.

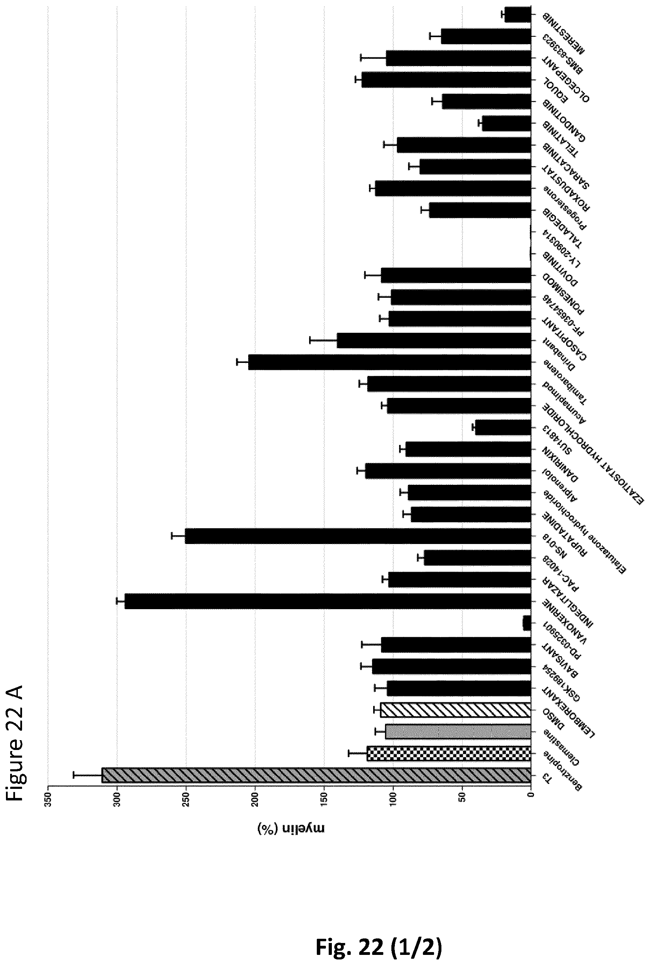

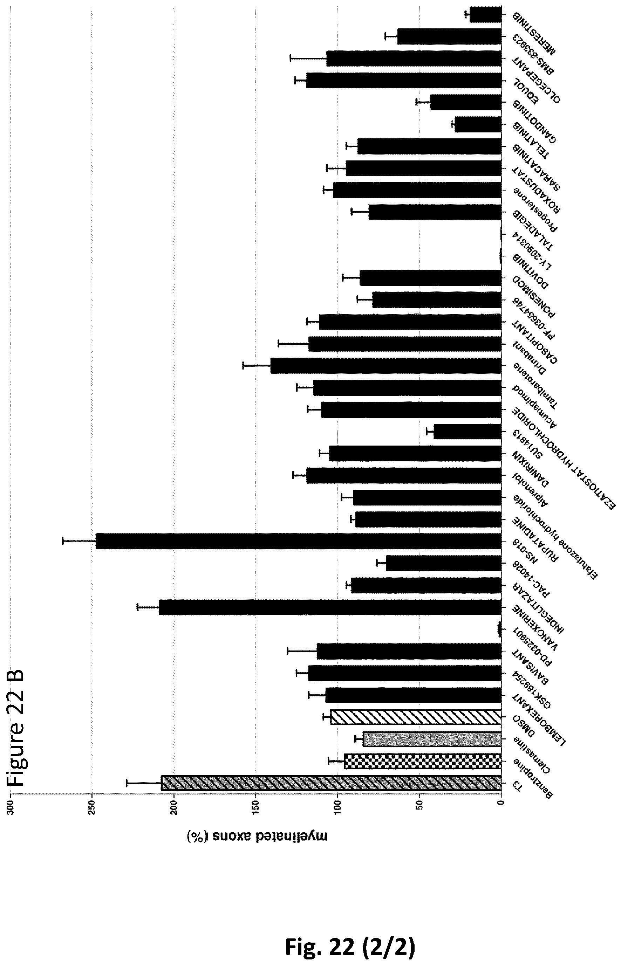

**Figure 22****. Evaluation of promyelinating capacity of 32 "Front Runners" compounds on primary murine spinal cord culture.** Primary cultures were treated with compounds at 1µM in "early treatment" paradigm at DIV 7-14, with readout of myelin area Figure 22 (A) and total area of myelinated axons, Figure 22 (B). Each bar represents mean ± standard error of the mean (SEM) value obtained from 5-10 wells (1-2 experiments).

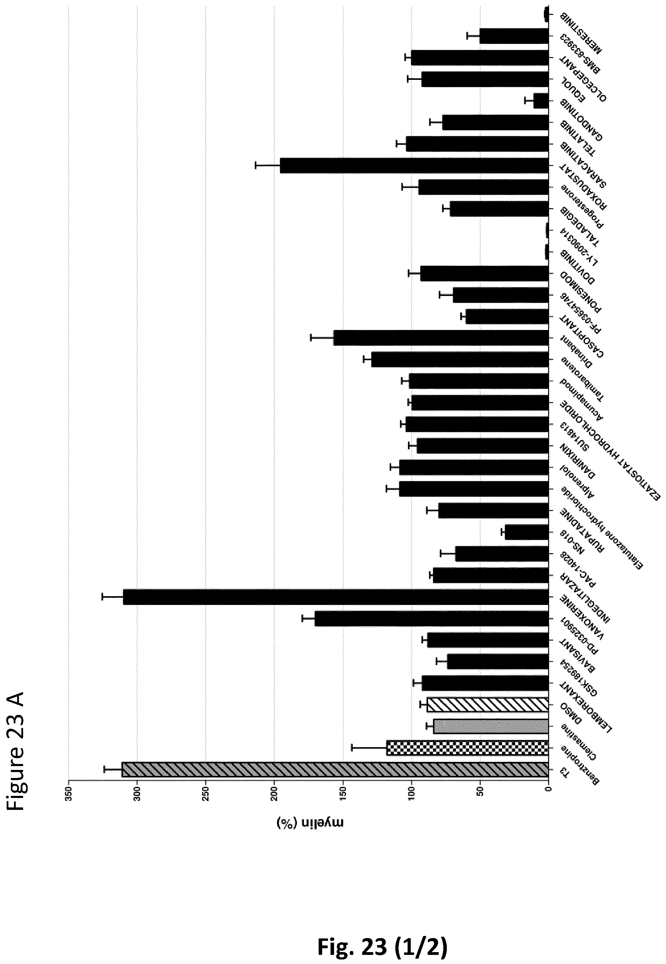

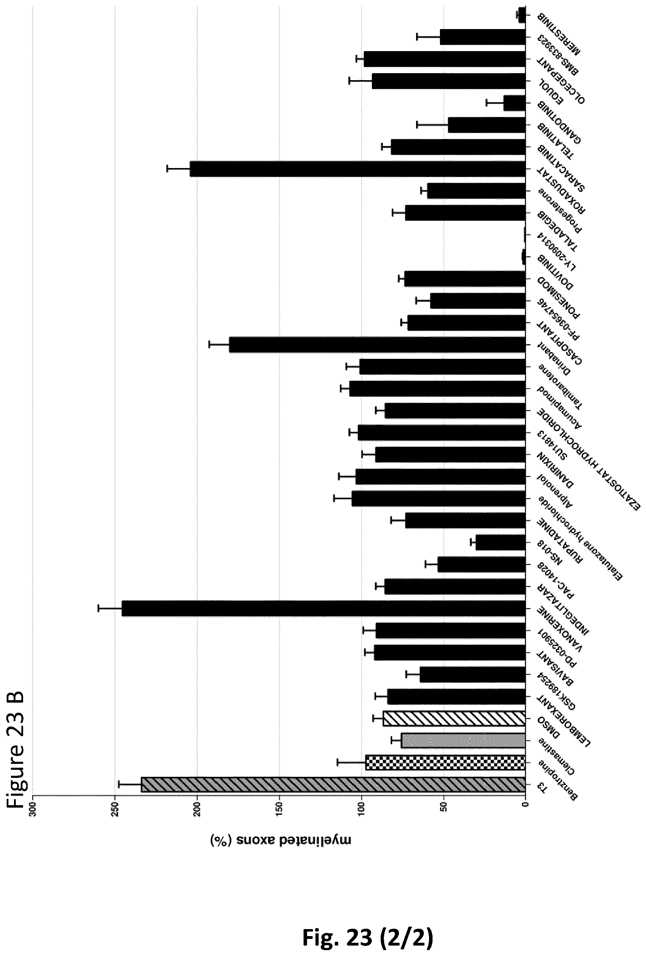

**Figure 23****. Evaluation of promyelinating capacity of 32 "Front Runners" compounds on primary murine spinal cord culture.** Primary cultures were treated with compounds at 1µM in "early treatment" paradigm at DIV 14-21, with readout of myelin area Figure 23 (A) and total area of myelinated axons, Figure 23 (B). Each bar represents mean ± SEM value obtained from 5-10 wells (1-2 experiments).

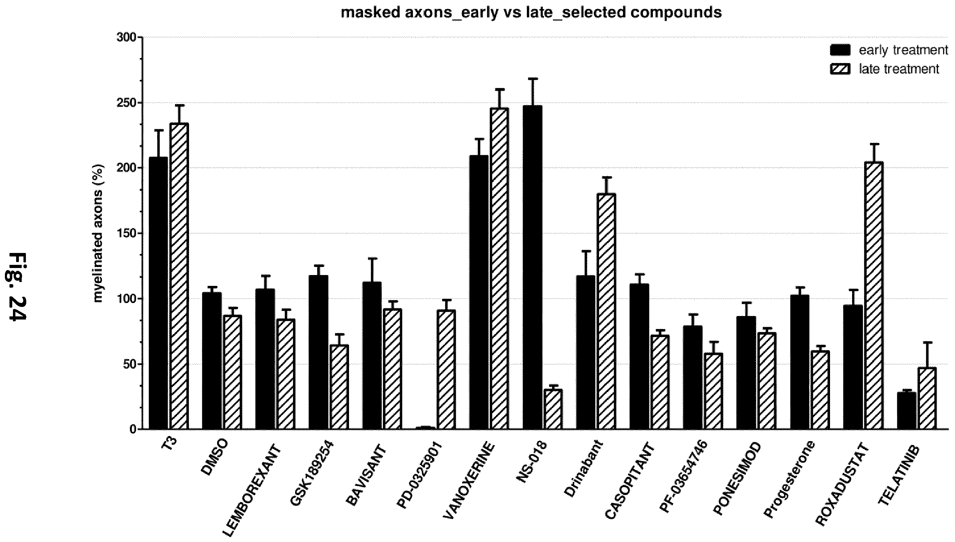

**Figure 24****. Comparison early vs. late treatment of selected compounds on primary murine spinal cord culture by quantification of myelinated axons.** Imaging data were analysed using CellProfiler and in-house generated analysis pipeline. Number of imaged wells (n = 5-10, 1-2 independent experiments). Values are depicted as mean ± SEM.

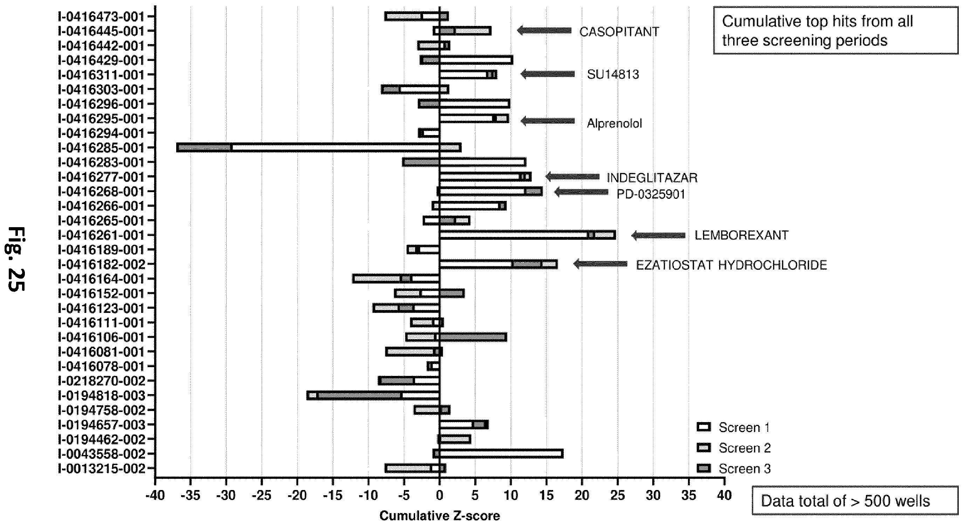

**Figure 25****. Screening for neurotoxic or regenerative effect on H9-derived human neural stem cells.** Cumulative Z-scores of three independent screenings for neurotoxic or regenerative effect of 32 selected compounds (Front Runners) on H9-derived human neural stem cells which identified 7 potential hits. Compounds were tested at 10 microM concentration. The assay readout is ATP production by Cell Titer Glow (Promega). The tested compounds: I-0416473-001 (MERESTINIB), I-0416445-001 (CASOPITANT), I-0416442-001 (PF-03654746), I-0416429-001 (DRINABANT), I-0416311-001 (SU14813), I-0416303-001 (ACUMAPIMOD), I-0416296-001 (DANIRIXIN), I-0416295-001 (ALPRENOLOL), I-0416285-001 (NS-018), I-0416283-001 (PAC-14028), I-0416277-001 (INDEGLITAZAR), I-0416268-001 (PD-0325901), I-0416266-001 (BAVISANT), I-0416265-001 (GSK189254), I-0416261-001 (LEMBOREXANT), I-0416182-002 (EZATIOSTAT HYDROCHLORIDE), I-0416164-001 (BMS-833923), I-0416152-001 (PONESIMOD), I-0416123-001 (GANDOTINIB), I-0416111-001 (TELATINIB), I-0416106-001 (ROXADUSTAT), I-0416081-001 (TALADEGIB), I-0218270-002 (SARACATINIB), I-0194818-003 (DOVITINIB), I-0194758-002 (EQUOL), I-0194657-003 (TAMIBAROTENE), I-0194462-002 (RUPATADINE), I-0043558-002 (VANOXERINE), I-0013215-002 (PROGESTERONE).

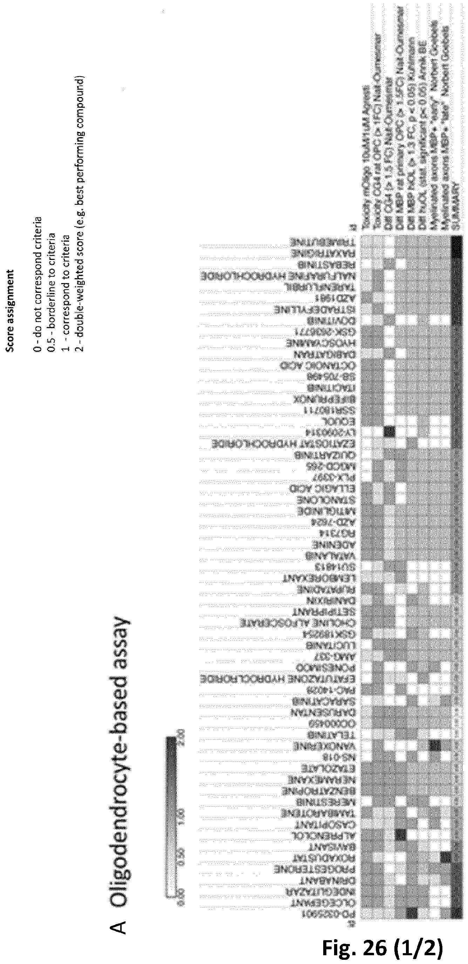

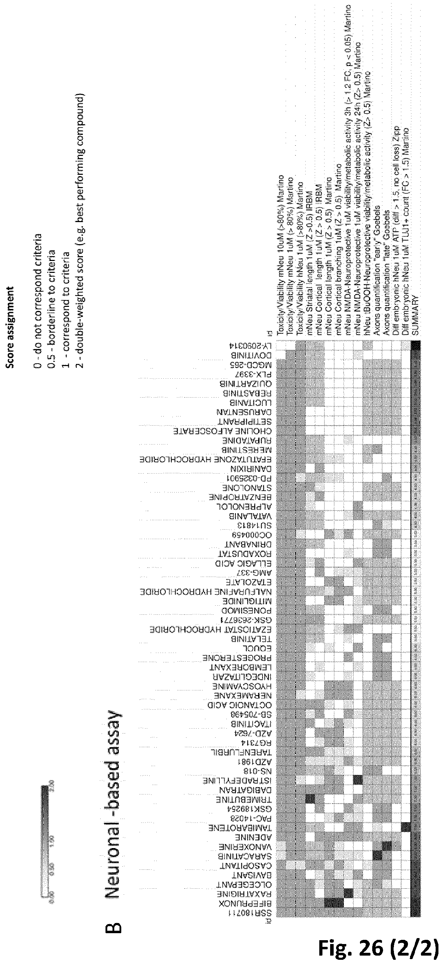

**Figure 26****. Heatmaps summary of oligodendrocyte-based and neuronal-based assays.** The color-code reflect the assigned scores corresponding to compound performance in selected assay, e.g., 0 - do not correspond to the criteria of the significance in assay; 0.5 - borderline to criteria; 1 - correspond to criteria; 2 - double-weighted score (hit-compound in assay).

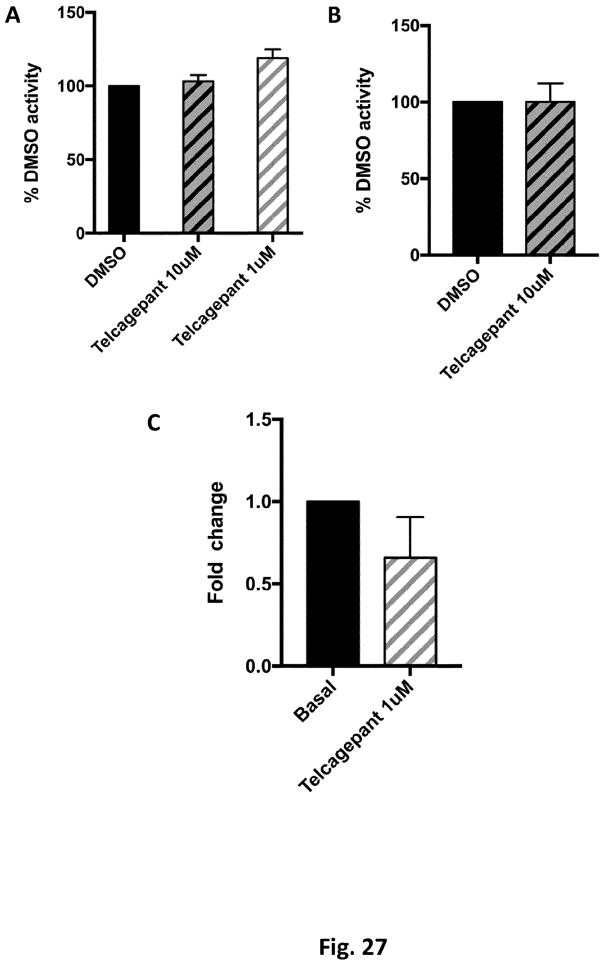

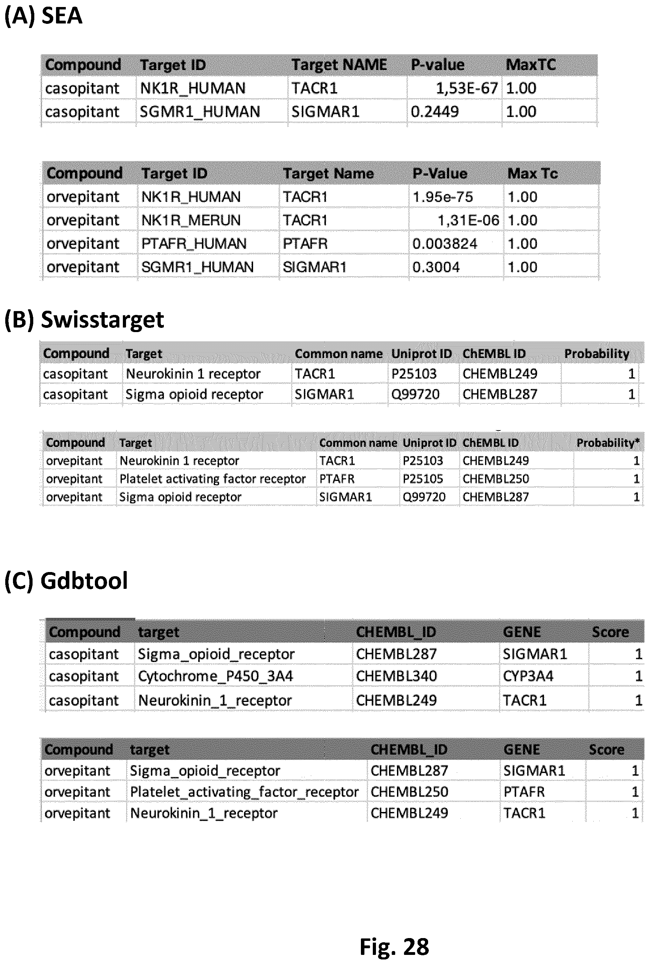

**Figure 27****. Telcagepant (1 and 10microM) is not toxic on oligodendrocytes.** Evaluation of telcagepant cytotoxicity on primary mouse cortical neurons (A), neonatal mouse oligodendrocyte progenitor cells (OPCs) (B), on CG4 line (C). In (A) and (B) the normalized viability is expressed with respect to the DMSO-treated control. In (C) the normalized viability is expressed with respect to the cells in basal condition. Each bar reports a mean value ± SD obtained from n =3 experiments run in triplicates. **Figure 28****. Predicted targets for Casopitant and Orvepitant.** Prediction of Casopitant and Orvepitant targets was evaluated by 3 different software: SEA (Keiser MJ. Et al Nat Biotech 2007), Swisstarget (Daina A. Nucleic Acids Research 2019) and Gdbtool (http://gdbtools.unibe.ch:8080/PPB/index.html). Only targets with the highest value (Score, MaxTC, Probability =1) are reported. Results indicate that Casopitant and Orvepitant bind TACR1 (also known as NK1R) and SIGMAR1. In addition, Orvepitant should also bind Platelet Activating Factor Receptor (PTAFR).

### MATERIALS AND METHODS

**Abbreviations: BDNF:** Brain-derived neurotrophic factor; **CNP:** 2'3'-cyclic nucleotide phosphodiesterase; **hiPSC:** human induced pluripotent stem cell; **OPC:** oligodendrocyte precursor; **OL:** oligodendrocyte; **MAP2:** microtubule-associated protein 2; **MBP:** myelin basic protein; **NAC:** N-acetylcysteine; **NMDA:** N-methyl-D-aspartate; **NMDAR:** NMDA receptor; **NPC:** neuroprecursor; **PBS:** phosphate-buffered saline.

### In silico platform to prioritize sets of therapeutic compounds that passed a safety assessment in man.

The *in silico* approach was based on the generation of biologically meaningful predictions of relationship integrating multiple high-throughput data sources. The approach computes features describing the network topology connecting two nodes. These features are used as input for a machine learning method that predicts the probability that an edge/connection exists. Evaluating the informativeness of each feature, the relevance of included domains can be compared providing insight into the influential mechanisms behind the process of interest for a given gene (http://het.io/hnep/). The predicted connectivity of one element of the network with others is weighted and ranked depending on the robustness of the connectivity. Het.io has been updated and expanded into SPOKE, as Scalable Precision medicine Open Knowledge Engine. For each gene/compound the number of established connections contributes to the calculation of the weighted score (DWPC score) (Figure 1). SPOKE has been further trained on medical records from the University of Southern California (UCSF) patient population (>300000 subjects) that spans 137 disorders. A self-organizing map (SOM) machine-learning algorithm is used to predict disease based on the training dataset of the UCSF population.

### Effect of compounds on the metabolism of neonatal mouse oligodendrocyte progenitor cells.

Purified oligodendrocyte progenitor cells (OPC) obtained from neonatal mouse primary mixed glial cultures²² were plated at the density of 6^{∗}10⁴ cells/cm² into poly-L-lysine-coated 96 well plates. One day after plating, the cells were incubated with or without drugs (10, 1 and 0.1µM) in DMSO (0.001% vehicle) for 48 hours. Compounds were screened for their ability to reduce [3-(4,5-dimethylthiazol-2-yl)-2,5-diphenyltetrazolium bromide (MTT)] using an automated microplate reader (Bio-rad, Hercules, CA, USA), as previously described.²² MTT (0.25 mg/ml, Sigma-Aldrich, St Louis, MO) was added to the culture medium during the final 4 hours of incubation. Edaravone (10 µM, Sigma-Aldrich) and platelet derived growth factor (PDGF, 20 ng/ml, Peprotech, Rocky Hill, NJ, USA) were used as positive controls due to their demonstrated activity on OPC proliferation and differentiation.²³ Only the compounds that showed an inhibitory effect on OPC metabolism at 10 µM were tested at lower concentrations.

### Differentiation of rodent oligodendrocyte progenitor CG4 line.

*Primary validation on CG4 line toxicity and differentiation.* An innovative OPC line was developed for fast and reliable high content screening (HCS) of small compounds inducing oligodendrocyte differentiation. The line was genetically engineered by lentiviral transduction of rat CG4 cells (PubMed=1613821; DOI=10.1002/jnr.490310125; Louis J.C., Magal E., Muir D., Manthorpe M., Varon S.; CG-4, a new bipotential glial cell line from rat brain, is capable of differentiating in vitro into either mature oligodendrocytes or type-2 astrocytes.J. Neurosci. Res. 31:193-204(1992) to express mCherry fluorescent reporter (Evercooren, Anne & Avellana-Adalid, V. & Vitry, Sandrine & Nait-Oumesmar, Brahim & Lachapelle, Francois. (1997). Expansion of Oligodendrocyte Progenitors for Myelin Repair. 10.1007/978-1-4615-5949-821) in mature oligodendrocytes only, and EGFP at all stage of the oligodendroglial lineage: both the generation of mature oligodendrocytes (wmN1-mCherry) and the morphological changes associated with differentiation (CMV-EGFP) can be therefore monitored in a single assay. mCherry expression occurred only in O4+/GalC+ differentiated oligodendrocytes and GFP was ubiquitously expressed at all developmental stages. The line so obtained was validated using compounds with well-established effects on oligodendrocyte differentiation (cAMP and T3). Cell-based assay was standardized on the HCS platform (ArrayScanXTI, Brain and Spine Institute (Paris), validating the pro-differentiation effects of clemastine²⁴ and 9cis-retinoic acid.²⁵ For HCS validation of 274 compounds identified through in silico screen, double-fluo CG4 line cells were plated in 96 well-plates, 6500 cells/cm² and left for 24h in proliferation. The other day, compounds were added (1µM) for next 5 days of differentiation, with 2/3 medium changed after 3 days. 9-cis Retinoic Acid (9cis-RA; 1µM) served as a positive control, negative controls were basal (N1) medium and vehicle (basal + 0.1% DMSO). Each compound was tested in triplicate and were imaged with ArrayScanXTI system (35 fields/well) after 5 days of differentiation. Compounds increased differentiation was evaluated by count of mCherry+ signal compared to basal and 9cis-RA controls, together with evaluation of number of cells (toxicity or proliferation).
*Secondary validation on primary rat OPC differentiation.* Re-validation of the best performing compounds promoting OPC differentiation (fold change > 1.5) was performed on primary rat OPC cultures in 96 well-plate run in triplicates by triple immunolabeling for CNPase, MBP and SOX10 as a readout. Compounds at 1µM were added for 4 days of differentiation and then cell cultures were imaged with ArrayScanXTI platform, number of MBP+ OLs were quantified by semi-automated software. Basal medium served as a negative control, 9 cis-RA (5µM) was a positive control.

### Differentiation of human iPS-derived neuroprecursors into oligodendrocytes.

NPC bearing a doxycycline inducible polycistronic construct containing SOX10, OLIG2 and NKX6.2²⁶ in a human safe harbor (passages 10 to 25) were singularized on day -3 by treatment with Accutase and plated on Matrigel-coated 12-well plates at a density of 100.000 cells per well in N2B27-medium containing equal parts of neurobasal (Invitrogen) and DMEM-F12 medium (Invitrogen) with 1:100 B27 supplement lacking vitamin A (Invitrogen), 1:200 N2 supplement (Invitrogen), 1 % penicillin/streptomycin/glutamine (PSG), 3 µM CHIR99021 (Axon Medchem), 150 µM ascorbic acid and 0.5 µM SAG. The next day medium was changed to N2B27-medium containing 1 µg/ml doxycycline. On D0 medium was changed to glial-induction medium (GIM) consisting of DMEM-F12 with 1:100 B27 supplement lacking vitamin A, 1:200 N2 supplement, 1 % PSG, 1 µM SAG, 10 ng/mL NT3 (Peprotech), 10 ng/mL IGF-I (Peprotech), 200 µM AA (Sigma), 1:1000 Trace Elements B (Corning). For full medium conditions 10 ng/ml T3 was added. On Day 2 medium was changed to glial-differentiation medium (GDM) comprising DMEM-F12 with 1:100 B27 supplement lacking vitamin A, 1:200 N2 supplement, 1 % PSG, 100 µM dbCAMP (Sigma), 100 µM AA, 1:1000. For full medium conditions 60 ng/mL T3, Trace Elements B 10 ng/mL, IGF-I, 10 ng/mL and NT3 were added to the culture. GDM was changed every other day. On day 7 cells were detached by treatment with Accutase and re-plated on laminin coated 48-well plates at densities of 10.000-12.000 cells per well. After 24 h cells were treated in minimal medium with either *vehicle* alone [0.01% (vol/vol) DMSO] as a negative control, 60 ng/mL T3 as a positive control, or with a drug candidate dissolved in DMSO. Medium was changed every other day. On D16 doxycycline was removed from the medium. Cells were fixed on D21 and analyzed via immunocytochemistry.

*Immunocytochemistry.* Cells were initially fixed with 4% PFA treatment for 20 min, RT. Following three washes with PBS cells were incubated with blocking solution containing 5% normal goat serum (NGS) and 5% FCS (fetal calf serum) in PBS for 30 min at RT to prevent unspecific binding of the antibodies. To detect intracellular antigens cells were permeabilized by adding 0.5% Triton X-100 to the Blocking-solution. Cells treated with Triton X-100 were washed three times with PBS each time for 5 min at RT. Primary antibodies (rat anti-MBP,1:50, Abcam, AB7349) were applied overnight, 4°C, in blocking solution. The next day cells were washed 3 times with PBS at RT, and afterwards incubated with secondary antibodies (AF488 Alexa Fluor anti-rat (goat), 1:500, Jackson/Dianova, #112-545-167) diluted in PBS for 1h at RT. Secondary antibodies were subsequently removed by washing 3 times with PBS. Afterwards PBS containing a Dapi staining for nuclei was added for 5 minutes and afterwards washed off with PBS. Cells were kept at 4°C until imaging.

*Imaging and evaluation.* From each condition (triplicates) 20-30 pictures were taken randomly chosen by the Dapi-signal. Cell numbers were determined by using an ImageJ Macro to count the nuclei whereas MBP positive cells were subsequently counted manually in black and white images.

### Human fetal neural precursors (hufNPCs) derived OPCs.

Fetal NPC²⁷ were expanded in Epithelial Growth Factor (EGF)+Fibroblast Growth Factor (FGF). At confluence, they were seeded in flasks and grown for 21 days in oligodendrocyte specification medium. At this stage cells were frozen and tested by immunohistochemistry (IHC): 70-80% were Platelet derived Growth Factor Receptor a (PDGFRa)+, 75% Oligodendrocyte Transcription Factor 2 (Olig2)+, 85% (sex determining region Y)-box 2 (Sox10)+ and 79% NK2 Homeobox 2.2 (Nkx2.2)+. Upon demand, frozen stocks were thawed for 2 days in the same medium before being switched for 7 days in 4 well plates to the oligodendrocyte differentiation medium containing either DMSO, Triiodothyronine (T3) or the compound (1µM) in 0.1% DMSO. Cells were then fixed and immunolabeled for Hoechst, Galactocerebrosidase (GalC) and anti-adenomatous polyposis coli clone CC1 (CC1).

The compounds selection was based on CG4/OPC/hiOL differentiation results (the most active and potentially new). Data were evaluated blindly and expressed as % of GalC+ OLs forming sheets vs CC1+ cells with compounds/DMSO at 7 days of differentiation.

### Differentiation of rodent primary neurons.

Primary cultures of murine cortical neurons were obtained from C57BL/6N female mice at gestation stage E16.5-E17.5. The cortices were washed 3 times with HBSS without phenol red (Hanks' Balanced Salt solution, Sigma) containing 5 mM HEPES (Sigma) and subsequently enzymatically dissociated for 30 minutes at 37°C, with one solution containing: trypsin 1.25 mg/ml (Sigma, dissolved in HBSS-phenol red, Euroclone), DNAsi 30 µg/ml (Sigma, dissolved in Neurobasal medium, Gibco), CaCl2 5mM (Sigma) in HBSS-phenol red. Afterwards, the enzyme activity was stopped using complete Neurobasal medium (Neurobasal medium, L-Glutamine (Invitrogen) 1%, penicillin-streptomycin 1%, B27 supplement (Invitrogen) 2%; FetalClone III Serum (FCIII) 10%, HyClone).

Cortical neurons were seeded at a density of 30,000 cells/ well in 96-well multi-well plate (Falcon) and 200,000 cells/coverglass in 24-well multi-well plate, coated with 100 µg/ml Poly-D-Lysine (Sigma) and 3.5 µg/ml laminin (Sigma) in PBS 1X. After 4 hours from the seeding the complete medium was replaced with serum-free complete Neurobasal medium. Cultures were maintained in a humidified CO2 incubator (5% CO2, 37°C) and half of the medium was changed every four days. The toxicity/neuroprotective assays were performed between 13-14 days in vitro (DIV). *Toxicity assay.* To evaluate toxicity of the molecules on murine cortical neurons, the 274 compounds have been tested in triplicates at 7-9 DIV in 96 multi-well plate at two pharmacological concentrations (1 µM and 10µM), with secondary cytotoxicity assay performed on more mature cultures at 14DIV. The cortical neurons were exposed to compounds for 24 hours and viability was assessed with Cell Counting Kit-8 assay (CCK-8, Sigma) compared to DMSO-treated cells.

### Neuroprotective assay.

To assess neuroprotective activity of 160 selected non-toxic compounds on primary murine cortical neurons, obtained as described in the previous paragraph, (13DIV), cultures were pre-treated with 1 µM compounds for either 3 or 24 hours, followed by 24 hours chronic stimulation with NMDA (8 µM) to induce neuronal damage. Each test plate included: a negative control (untreated), vehicle-treated control (0.02% vol/vol DMSO), a positive neurotoxic control - staurosporine 0.1 µM, a stressor - NMDA 8 µM, a positive neuroprotective control - NAC 300 µM, and a combination of stressor + neuroprotective molecule (NMDA+NAC). Cell viability was measured with Cell Counting Kit-8 assay compared to NMDA-treated control.

### Morphological integrity evaluation.

Apart of viability/metabolic readout in NMDA-mediated neurotoxicity assay, a neuronal structural integrity was evaluated by MAP2 staining. Mouse cortical neurons (14DIV) cultured in MW96 plates were fixed with 4% paraformaldehyde (PFA). Fixed cells were incubated with blocking solution (PBS IX, donkey serum 5%, Triton 0.1%) for 1-hour, primary antibody (rabbit α-MAP2, 1:250, Millipore) was applied in the same solution overnight at +4°C. The next day cells were washed 3 times with PBS 1X + Triton 0.1% at RT, and afterwards incubated with secondary antibody (Alexa Fluor 555 anti-rabbit (donkey), 1:1000, Molecular Probes, ab150074) diluted in PBS 1X + Triton 0.1% for 1h at RT. Nuclei were stained with 4'-6-diamidino-2-phenylindole (DAPI, Roche). Microscopy was performed using ArrayScan^{™}XTI (ThermoFisher) imaging platform. 15 images per well were acquired and analyzed by CellProfiler. Total neurite length and the total number of non-trunk branches were analyzed. Total neurite length and the total number of branching were evaluated from 15 images/well, each compound or control was tested in triplicates. The total neurite length and total number of branches were converted to a standardized Z-score using formula: Xᵢⱼ = ((Xij - µ)/*σ*), where xij is the raw measurement of the compound located in well (*i, j*), and µ and σ are, respectively, the mean and the standard deviation of all measurements of the plate.³¹

***Morphological integrity evaluation.*** Mouse cortical and striatal neurons (14DIV) cultured in MW96 plates were exposed to 1uM to 60uM concentrations of NMDA and relationship between the NMDA dose and fiber length was evaluated. To assess neuroprotective activity of 114 selected non-toxic compounds on primary murine cortical and striatal neurons (14DIV), cultures were pre-treated with 1 µM compounds for 24 hours, followed by 24 hours chronic stimulation with NMDA (20 µM) to induce neuronal damage. Each of the compound was tested in quadruplicate. The fiber length was evaluated by MAP2 staining (please see the method described earlier).

### hiPSC generation.

Skin biopsies from 6 twin pairs (MS patients and healthy controls) discordant for disease have been collected. Skin biopsies were first divided in pieces and then cultured in a sterile serum rich medium until fibroblasts spread from the tissue and grown exponentially. Human fibroblasts were maintained in DMEM containing 10% FBS, 2 mM L-glutamine, 1x10⁻⁴ M nonessential amino acids, 1 mM sodium pyruvate and 0.5% penicillin-streptomycin. The explants were screened for presence of mycoplasma using a standard PCR kit. Fibroblasts were expanded and cryopreserved at 1-2 million cells per vial in FBS and 10% DMSO or seeded immediately for reprogramming. For iPSCs generation, fibroblasts within fourth passages were used to avoid replicative senescence.

For hiPSC generation, fibroblasts were infected with the CytoTune^{™}-iPS 2.0 Sendai Reprogramming Kit (Life Technologies). After 1 week, the transduced fibroblasts were plated on a feeder layer of mitotically inactivated (mitomycin C (Sigma Aldrich) MEFs (2.5 × 10⁴ cells/cm²) in a DMEM supplemented with 20% KSR, 2 mM L-glutamine, 0.1 mM nonessential amino acids, 1 mM sodium pyruvate, 0.02 mM β-mercaptoethanol, 1% penicillin-streptomycin, and 10 ng/ml FGF2. Medium was changed every day and colonies started to appear 3-4 weeks later. After approximately one month, colonies with the correct morphology were picked and transferred to a Matrigel coated (feeders free) culture in mTeSR^{™} (StemCell Technologies) medium. Colonies that reached at least passage 5 were assessed: for the absence of Sendai virus via qRT-PCR, their effective pluripotency (expression of pluripotency markers OCT4, NANOG, SOX2 evaluated by immunocytochemistry and qRT-PCR), and for their ability to differentiate into the three main germ layers via embryoid bodies formation (differentiation to mesoderm (dME), endoderm (dEN), and neuroectoderm (dEC) potential).

### hiPSC differentiation into neural progenitors (NPCs) and neurons.

This protocol was modified from Yuchen Qi et al. ²⁸ First, neural progenitors (NPCs) were generated from hiPSCs by dual-SMAD inhibition. Shortly, at day 0 iPSC colonies (confluent at 70-80%) were washed with PBS Dulbecco's w/o Calcium w/o Magnesium (Euroclone) and hiPSCs' maintenance medium (mTeSR^{™}, StemCell Technologies) was replaced with neural induction medium composed by DMEM/F12 plus N2 medium (1X), B27 without RA supplement (0.5X) (Life Technology), added with SB431542 (10µM) and LDN (500nM). This medium was kept until day 4 ("induction"). At day 4, cells were splitted (1:3) with the same induction medium using Accutase^{®}/Accumax^{™} (StemCell Technologies) dissociated solution. Entire medium was replaced daily up to days 14-16 until rosette formation was observed ("patterning"). At days 14-16, cells were detached by Accutase^{®}/Accumax^{™} and re-plated on polyornitine/laminin/fibronectin coated MW96 or MW24 plates for terminal differentiation in Neurobasal medium supplemented with B27 (0.5x), BDNF (20ng/ml), dbcAMP (500µM), ascorbic acid (200 µM), PD0325901 (1 µM), SU5402 (5 µM), CHIR (3 µM), human recombinant insulin (5 µg/ml), supplemented with RI (Rock inhibitor) (1:3000) to reduce dissociation induced apoptosis. The half of differentiation medium was changed every 3-4 days, supplemented with 1 µg/ml laminin and DAPT (1-2 µM) until terminal maturation of neuronal population (MAP2+cells). Cultures were characterized by immunocytochemistry and qRT-PCR and neuroprotective experiments were performed on days 30-40 of differentiation.

### ROS-mediated and neuroprotective assay on human iPSC-derived neurons (hNeu).

Neuroprotective experiments were performed using iPSC-derived glutamatergic neurons from 3 healthy controls cultured for 30-40 DIV. Cells were pre-treated for 24h with 1µM of compounds, vehicle (DMSO) or 5nM Mito-Q (mitoquinone) served as a positive control. On the next day cells were incubated for 2h with 100µM of tert-butyl peroxide (tBuOOH, Sigma), then the medium was replaced and the viability readout by CCK8 viability assay kit (Dojindo) was performed on the following day. The cell viability was expressed as normalized Z score and compared to tBuOOH-treated cells with nonparametric Kruskal-Wallis test with Dunn's multiple comparisons correction.

### Human embryonic and fetal neural precursors (hufNPCs) derived neurons.

Neural progenitors were derived from a single human fetus, and a non-immortalized human neural progenitor cell (hNPC) line was obtained and maintained in chemically defined serum-free growth medium (containing FGF-2 and EGF) as described in detail below.

Primary, growth factor-expanded hNPCs were obtained as a heterogeneous culture of spherical cell aggregates, derived from the diencephalic and telencephalic regions of a single human (Caucasian male fetus at 10-12 weeks gestational age (#BI-0194-008), obtained from a pregnancy interruption). Cells were cultured in stationary conditions both in T flasks as well as in cell factories in non-GMP conditions. Human tissue was provided by Banca Italiana del Cordone Ombelicale Fondazione IRCCS CA' GRANDA Ospedale Maggiore Policlinico in Milan.

First, fresh human fetal brain tissues were mechanically dissociated, followed by enzymatic dissociation with Trypsin (LONZA (BE17-161E) 1:5) in growth medium for 5-10 min at 37°C, 5% O2 e 5%CO2, then washed with 10% Australian FBS in fresh medium and centrifuged 15 min at 200 g. Cells were plated in T25 flask in Neural stem cell growth medium (NeuroCult-XF Basal Medium (cat.# 05760)+ NeuroCult- XF Proliferation supplement (cat.# 05763) (for Human Neural Stem Cells) STEMCELL TECHNOLOGIES + human recombinant carrier-free EGF (cat. # 1325 9510 00) and bFGF (cat. # 1370 9505 00) (Provitro) at final concentration of 20 ng/ml) at 37°C, 5% O2 e 5% CO2. After 15-25 days, enzymatic (Accumax^{®}, StarFish cat. # GMP-102470) dissociation of neurospheres or subconfluent adherent cells was performed and cells were re-plated at density (25000 cells/cm2) in NGM. Every 15 days the cells were passaged, expanded and cryopreserved. This cell line represents a stable and renewable source of uncommitted hNPCs that can be safely expanded, differentiating spontaneously versus glial and neuronal progeny when exposed to growth factor - free medium. Cell viability evaluation, mycoplasma presence (PCR), karyotype analysis and immunocytochemistry (b-tubulin, GFAP, O4) were used for characterization. Pro-differentiating properties of 160 compounds were evaluated by co-incubating freshly seeded in 96 well plate hufNPCs with 1 µM compounds for 7 days (each compound was tested in triplicate). 0.02% vol/vol DMSO and basal medium served as negative controls, heparin²⁹ served as a positive control. After one week, cells were fixed and analyzed via immunocytochemistry. Immunocytochemistry was performed as described above: cells were immunolabeled for TUJ1 (mouse α-TUBB3, 1:1000, BIOLEGEND, 801202) and GFAP markers (rabbit α-GFAP, 1:500, DAKO, Z0334). Nuclei were stained with 4'-6-diamidino-2-phenylindole (DAPI, Roche). Microscopy was performed using ArrayScan^{™}XTI (ThermoFisher) imaging platform. 15 images per well were acquired and analyzed by CellProfiler. Number of TUJ1+/DAPI cells was counted and compared to DMSO.

### Myelinating cell cultures from primary murine spinal cord.

Single cell suspensions are prepared from spinal cord of mouse embryos (E13) (adapted from Thomson, 2008) and 1.5 x 10⁵ cells/well were plated into 96 well plates. Over time, these cultures mature and form axons, which later myelinate. Using spinal cord tissue facilitates the quantification of mature MBP+ oligodendrocytes and newly formed myelin by in-house high-content imaging system (Operetta High Content Imaging System, Perkin Elmer) with a 20x lens. From each well, 25 fields in a 5x5 matrix were scanned. Thirty-two "Front Runners" compounds were tested in replicates of five at 1 µM /DMSO, while T3, benzotropine and clemastine were used as positive controls, DMSO as negative/solvent control. Two experimental paradigms were applied: "Early treatment": exposure to experimental agent from DIV7 to DIV14; "Late treatment": exposure to experimental agent from DIV14-DIV21. On DIV21, cultures were fixed and stained (myelin: MBP, axons: NF200; nuclei: Hoechst). Imaging data were analysed using CellProfiler and in-house generated analysis pipeline.

### Screening for neurotoxic or regenerative effect of compounds on H9-derived human neural stem cells.

H9-derived human neural stem cells (H9 hNSCs, Gibco) were cultured according to the manufacturer's instructions. Briefly, H9 hNSCs were expanded and subsequently differentiated in neurobasal medium, 2% B27, 1% Glutamax, and 1% penicillin/streptomycin, supplemented with 10 ng/ml BDNF (Peprotech), and 2 ng/ml recombinant human GDNF (Peprotech). After 7 days, 500 µM of db-cAMP (N6,2'-O-dibutyryladenosine 3',5'-cyclic monophosphate, Sigma) was added.

The metabolic activity/viability of selected compounds (32 "Front Runners" + 24 compounds from the "Short list") was evaluated by CellTiter-Glo^{®} Luminescent Cell Viability Assay. The primary screen of Front Runner List (32 compounds) + 24 compounds from Short List) on H9-derived human neurons was performed at 10µM concentration. The validation of hits from primary screen on H9-derived human neurons was performed at 10µM concentration.

### RESULTS

Based on the clinical and biological evidence available for PMS, authors tested if directly or indirectly favoring neuroprotection could prevent disease progression. Promoting resistance to axonal degeneration and increasing neuronal survival in demyelinating conditions can extend the period in which axons can be remyelinated. On the other side, promotion of oligodendrocyte proliferation and differentiation is a valuable alternative because indirectly favors neuroprotection.

To accomplish these goals, molecules of interest were prioritized *in silico,* and then analyzed in appropriate phenotypic assays.

The methodological approach consists of starting with a large portfolio of repurposed or abandoned molecules (1500) that have been screened *in silico.* Selected molecules (511) have been transferred to large and mid-scale screening (hit identification). Thirty-nine selected molecules passed stepwise functional assays in oligodendrocytes and neurons, which provided validated data supporting a neuroprotective and/or remyelinating therapeutic effect.

### 1. In silico platform to prioritize sets of therapeutic compounds that passed a safety assessment in man.

To bioinformatically approach Progressive MS, a selection of keyworks have been used as input for SPOKE, such as myelin formation, oligodendrocyte differentiation, myelin, etc. An enriched list of 511 compounds with a DWPC < 0.0005 has been generated starting from the entire SPOKE network that include 1,941,858 nodes of 12 types. The list of compounds was analyzed to remove redundant items because of structure chemical similarity and to run predictive *in silico* pharmacological strategies to assess whether those compounds could pass the blood brain barrier (BBB).

### 2. Oligodendroglia-based assays

### 2.1 Toxicity assays on neonatal mouse oligodendrocyte progenitor cells.

Toxicity of the full list of drugs (274) at 10µM have been evaluated on neonatal mouse oligodendrocyte progenitor cells via metabolic activity by MTT. First, drugs able to reduce OPC viability were selected, identifying 90 compounds with a cytotoxic effect defined by the Efficacy Ratio ER (absorbance of drug/absorbance of vehicle; PMID: 28387380) (ER ≤ 0.4) which have not been further investigated - and 44 drugs inhibiting OPC metabolism at lower level (0.5<ER<0.8) - which were analyzed in the next experimental setting. As a result, 90 compounds were cytotoxic, 44 compounds with low toxicity were retested at 1 and 0.1 µM, 140 compounds (ER > 0.8) were non-toxic and were re-tested. Results are shown in Figures 1 and 2.

### 2.2 Toxicity assays on rat oligodendrocyte progenitor CG4 line.

Compounds (272) were tested at 1 µM, using a double fluorescence CG4 cell line (rat OPC line), 9 cis-retinoic acid (1µM) served as a positive control. Toxicity was estimated, based on the compounds' effect on cell density: 32/272 (11.7%) compounds promoted OPC proliferation (fold change > 1.5), 126/272 (46%) resulted in no toxicity (1< fold change < 1.5), 75/272 (27.5%) were moderately toxic (0.5 < fold change < 1.0), while 39/272 (14.3%) exhibited high toxicity (fold change < 0.5). Results are shown in Figure 3.

### 2.3 Differentiation assays on rat oligodendrocyte progenitor CG4 line.

Figure 4 provides a schematic representation of High Content Screening (HCS) validation of the 274 compounds identified through *in silico* screening using CG4 line. 274 tested compounds were also classified according to their ability to induce OPC differentiation: 226/274 (82.5%) compounds did not have effect on OPC differentiation, 21/274 (7.7%) compounds had positive effect but potential toxicity, while 27/274 (9.8%) compounds promoted OPC differentiation and exhibited a low toxicity (mCherry+OLs plus compounds/mCherry+ OLs plus N1 medium) (Figure 5). In addition, the whole short list of 160 non-toxic compounds were screened using the CG4 high content phenotypic assay to further confirm the data from primary screening and to potentially select additional compounds with pro-myelinating activities. From this screening of the short list, 49 hit compounds were identified that increase differentiation either of CG4 cells or primary OPCs (Figure 6). These 49 hits include all the front-runner list of compounds (32), which were initially identified in the first screen, thus demonstrating the robustness of this phenotypic assay. Importantly, 14 additional compounds that exhibited a strong effect on CG4 differentiation (over 1.5-fold increase relative to control) were selected, among which benztropine was also identified.³⁰

### 2.4 Differentiation of rat primary OPC.

Re-validation of the 49 compounds selected from the short list (160 compounds) using rat primary OPC cultures (compounds tested in triplicates in 3 independent experiments. The secondary validation screen of these compounds performed on rat primary OPC cultures selected 17 best lead compounds with a pro-differentiation activity (MBP+/SOX10+ OLs plus compounds/MBP+SOX10+ plus basal medium fold increase) induced a strong differentiation into MBP+ OLs (fold increase >1.6). Results are shown in Figure 7.

### 2.5 Differentiation of human iPS-derived neuroprecursors into oligodendrocytes.

The effect on oligodendroglial differentiation of 32 compounds ("Front runners") was tested. Induced pluripotent stem cells in which the transcription factors SOX10, OLIG2, NKX6.2 were inserted in a doxycycline inducible manner into a human safe harbor were differentiated into neural stem cells. Differentiation into oligodendrocytes was induced by addition of doxycycline. Cells were cultured either in a minimal medium (MM) (negative control) or in MM with the different compounds at a concentration of 1 µM. Addition of T3 to the minimal medium served as a positive control. The following compounds were able to promote significantly the differentiation into MBP+ oligodendrocytes: Drinabant, Roxadustat, Gsk189254, Casopitant, Saracatinib, Bavisant, PF-03654746, Telatinib, PD-0325901, Danirixin, Ponesimod, Indeglitazar, Ezatiostat hydrochloride, Olcegepant, NS-018, Progesterone and Merestinib. Results are shown in Figure 8.

### 2.6 Human fetal neural precursors (hufNPCs)-derived OPCs differentiation.

16 compounds were evaluated on human fetal neural precursors (hufNPCs) differentiation and identified 4 compounds that significantly promoted fNPCS differentiation (fold change>1.5, p<0.05). These compounds were Olcegepant, Merestinib, Indeglitazar, Efatutazone hydrochloride. In addition, four compounds namely Bavisant, Telatinib, PD0325901 and Drinabant promoted fNPCS differentiation with moderate significance (fold change>1.3, 0.05<p<0.07). Results are shown in Figure 9.

### 3. Neuronal-based assays

Schematic representation of neuronal-based assay screening strategy is shown in Figure 10.

### 3.1 Characterization of rodent primary neurons

Primary cultures of murine cortical neurons were established from E16.5-E17.5 embryos and characterized by immunofluorescence at DIV 7 and DIV 14 for expression of neural (MAP2), pre-synaptic (vGLUT1 and NMDAR1) markers (Figure 11). Cells expressing the vesicular glutamate transporter 1 (vGLUT1) were already present at DIV7, increasing through DIV14, indicating the differentiation into glutamatergic neurons.

### 3.2 Evaluation of compound cytotoxicity on primary murine cortical neurons.

Neural toxicity of 274 compounds have been evaluated: 145/274 (53%) at 1 µM (FIG. 12, E) and 97/274 (35.4%) at 10 µM (FIG12, F) promoted cell metabolic activity, while 28/274 (10.2%) compounds were cytotoxic at both concentrations based on tetrazolium assay (CCK8), (FIG12, D). Based on cytotoxicity results obtained on mouse cortical neurons and mouse forebrain oligodendrocytes, 160/272 non-cytotoxic compounds were further selected for evaluation of neuroprotective potential. In addition, 47 compounds were re-tested in duplicate to evaluate the reproducibility of toxicity at 1 µM (FIG12, A) and 10 µM (FIG12, B).

### 3.3 Evaluation of compounds neuroprotective potential.

The potential neuroprotective activity of 160 selected non-toxic compounds was evaluated on murine cortical neurons (14DIV) with either 3h (Figure 13) or 24h compounds pre-treatment (Figure 14). In assay with 3h pre-treatment, 17 compounds were identified as potential NMDA-neuroprotective hits (marked with black asterisk in Figure 13, stat. significant at p<0.05, Kruskal-Wallis test with post-hoc two-stage linear step-up procedure of Benjamini, Krieger and Yekutieli multiple comparison correction).

The identified 17 hit compounds were: Tamibarotene, Progesterone, SSR180711, Adenine, GSK189254, Neramexane, Raxatrigine, OC000459, AMG-319, Hyoscyamine, SB-705498, AZD1981, Ezatiostat hydrochloride Nalfurafine hydrochloride, Pamapimod, Fluocinolone acetonide, L-Serine. The additional potentially NMDA-neuroprotective compound that had a trend versus significance was Tarenflurbil (p=0.09).

In assay with 24h pre-treatment, we have identified 32 compounds with a potential neuroprotective capacity against NMDA-induced excitotoxicity with Z>0.5: Istradefylline, Vatalanib, Trimebutine, Alprenolol, Ellagic Acid, SSR180711, Tamibarotene, Adenine, Ezatiostat hydrochloride, Bavisant, Equol, Etazolate (all aforementioned are shown in green in FIG.14), while other identified compounds such as Benztropine Mesylate, Encenicline, Tenofovir, SDX-101, APD334, GS-4997, Doramapimod, Vidupiprant, L-Serine, Seliciclib, Velneperit, Netoglitazone, Prinaberel, PF-03654746, Flindokalner, Elinogrel, Raseglurant, Taladegib, L-Phenylalanine, Talmapimod are not shown in Figure 14.

### 3.4 Morphological integrity evaluation.

The morphological integrity of mouse cortical neurons upon chronic 24h NMDA (8µM) exposure with compounds pre-treatment (3h) in preventive regimen was evaluated. Control (basal), DMSO, N-acetylcysteine (NAC) served as positive controls, while staurosporine and NMDA were negative controls.

38 compounds scored Z>0.5 in neurite length evaluation and 29 compounds scored with Z>0.5 in branching evaluation. 26 compounds that preserved both neurite length and the network integrity (branching) were: Bifeprunox, SSR180711, Mitiglinide, AZD-7624, Olcegepant, RG7314, Neramexane, Raxatrigine, Adenine, Hyoscyamine, Etazolate (Shown in Fig 15), while compounds Ibipinabant, L-Phenylalanine, Tideglusib, Basimglurant, Indiplon, Velneperit, GS-4997, Defactinib, Capmatinib, Varlitinib, Seliciclib, Orteronel, Oliceridine, Ramatroban, VX-702 are not shown. The microscopic images of the two best-performing compounds identified in this assay Bifeprunox and SSR180711 are showed in Figure 16.

42 compounds were screened to evaluate the morphological integrity of cortical and striatal neurons upon chronic (24h) NMDA exposure at concentration of 20µM with compounds pre-treatment (24h) in preventive regimen (Figure 17). 10 compounds were selected for confirmation, namely: Trimebutine (hit-compound in both cortical and striatal neuronal assays), AMG-208, Flindokaliner, GSK-2636771, Tarenflurbil, SSR180711, Emixustat HCL, Bifeprunox, Olcegepant, Casopitant.

### 3.5 Differentiation of human neural stem cell and human iPSC derived NPCs.

The scheme for the generation of glutamatergic neurons from iPSC is shown in Figure 18. hiPSCs were obtained by reprogramming from skin fibroblasts with a small-molecule approach. iPSCs were further differentiated into neural precursor cells (NPC), and further into glutamatergic neurons.

Evaluation of cytotoxicity of 160 selected compounds on iPSC-derived neurons (DIV45). Cytotoxicity of selected 160 compounds was evaluated on iPSC-derived neurons (45DIV) from a healthy control line: 12/160 compounds exhibited moderate cytotoxic effect (∼65-80% viability compared to DMSO), namely BMS-833923, PD-0325901, Casopitant, Zibotentan, Istradefylline, OC000459, K-877 Pemafibrate, Reminertant, Derenofylline, Basimglurant, Neramexane. Results are shown in Figure 19.

### 3.6 ROS-mediated neuroprotective assay on hNeu.

32 "Front runners" and 7 compounds from short list were tested on iPSC-derived neurons from three control lines in ROS-mediated assay to evaluate a neuroprotective potential of selected compounds. Three independent experiments were performed for CTR4 and CTR8, Figure 20 (A, C), while two independent experiments were performed for CTR7, Figure 20 (B). The summary of all experiments for all three control lines are shown in Figure 20 (D). For CTR4, we identified 4 compounds protected from tBuOOH insult, (stat. significantly p<0.05), namely GSK189254, NS-018, Casopitant, Equol. For CTR7, only Casopitant was identified with a trend to significance (p = 0.07). For CTR8, NS-018 was identified as a potential neuroprotective compound from tBuOOH-mediated insult (p<0.05).

As a summary for all three control lines, 5 compounds have been identified with a potential neuroprotective effect from tBuOOH-mediated insult, such as NS-018, p<0.0001; Casopitant, p<0.0001; Saracatinib, p=0.0007; Ezatiostat HCl, p=0.002; Olcegepant, p=0.012.

### 3.7 Human embryonic and fetal neural precursors (hufNPCs) derived neurons.

160 selected non-toxic compounds pro-differentiating properties were evaluated on human embryonic and fetal neural precursors (hufNPCs). Assay was performed twice and run in triplicates in 96-well plates, the compound performance was compared to DMSO. Hit-compound Tamibarotene (Retinoic acid receptor alpha/beta agonist) was identified with significantly promoted neuronal differentiation compared to DMSO and heparin.²⁹ In addition, other seven compounds with a potential neural pro-differentiating properties were identified, among which were LY-2090314 (Glycogen synthase kinase 3 beta (GSK3) inhibitors)³², Doramapimod, Acumapimod, Losmapimod (all three compounds are P38 mitogen-activated protein kinase inhibitors)³³, Marimastat (Metalloprotease inhibitors), Rupatadine (Histamine H1 receptor antagonists) and Quizartinib (Fms-like tyrosine kinase 3 inhibitors). Results are shown in Figure 21.

### 3.8 Myelinating cell cultures from primary murine spinal cord.

Thirty-two "Front Runners" compounds were tested in replicates of five at 1 µM /DMSO, while T3, benztropine and clemastine were used as positive controls, DMSO as negative/solvent control. Two experimental paradigms were applied: "Early treatment": exposure to experimental agent from DIV7 to DIV14; "Late treatment": exposure to experimental agent from DIV14-DIV21. In "Early treatment" paradigm in myelin area evaluation were identified Vanoxerine, NS-018, Tamibarotene, Progesterone, Ponesimod, Casopitant, PF-0365476, Drinabant, Saracarinib. In quantification of myelinated axons were identified Vanoxerine, NS-018, Casopitant. In "Late treatment" paradigm in myelin area evaluation were identified Vanoxerine, Roxadustat, Tamibarotene, Drinabant, Progesterone, Saracatinib. In quantification of myelinated axons were identified Vanoxerine, Drinabant, Roxadustat.

### 3.9 Screening for neurotoxic or regenerative effect of compounds on H9-derived human neural stem cells.

32 Front Runners were tested at 10µM on H9-derived human neuronal cultures and viability was evaluated by CellTiter-Glo^{®} Luminescent Cell Viability Assay. Cumulative Z-scores of three independent screenings for neurotoxic or regenerative effect identified 7 potential hits, namely Casopitant, SU14813, Alprenolol, Indeglitazar, PD-0325901, Lemborexant and Ezatiostat Hydrochloride.

The tested compounds were: I-0416473-001 (MERESTINIB), I-0416445-001 (CASOPITANT), I-0416442-001 (PF-03654746), I-0416429-001 (DRINABANT), I-0416311-001 (SU14813), I-0416303-001 (ACUMAPIMOD), I-0416296-001 (DANIRIXIN), I-0416295-001 (ALPRENOLOL), I-0416285-001 (NS-018), I-0416283-001 (PAC-14028), I-0416277-001 (INDEGLITAZAR), I-0416268-001 (PD-0325901), I-0416266-001 (BAVISANT), I-0416265-001 (GSK189254), I-0416261-001 (LEMBOREXANT), I-0416182-002 (EZATIOSTAT HYDROCHLORIDE), I-0416164-001 (BMS-833923), I-0416152-001 (PONESIMOD), I-0416123-001 (GANDOTINIB), I-0416111-001 (TELATINIB), I-0416106-001 (ROXADUSTAT), I-0416081-001 (TALADEGIB), I-0218270-002 (SARACATINIB), I-0194818-003 (DOVITINIB), I-0194758-002 (EQUOL), I-0194657-003 (TAMIBAROTENE), I-0194462-002 (RUPATADINE), I-0043558-002 (VANOXERINE), I-0013215-00 2 (PROGESTERONE).

### REFERENCES

1. Olesen J, Gustavsson A, Svensson M, Wittchen HU, Jönsson B. The economic cost of brain disorders in Europe. Eur J Neurol. 2012;19(1):155-162. doi:10.1111/j.1468-1331.2011.03590.x
2. Zaratin P, Battaglia MA, Abbracchio MP Nonprofit foundations spur translational research. Trends Pharmacol Sci. 2014;35(11):552-555. doi:10.1016/j.tips.2014.09.003
3. Adelman G, Rane SG, Villa KF. The cost burden of multiple sclerosis in the United States: A systematic review of the literature. J Med Econ. 2013;16(5):639-647. doi:10.3111/13696998.2013.778268
4. Hartung DM, Bourdette DN, Ahmed SM, Whitham RH. The cost of multiple sclerosis drugs in the US and the pharmaceutical industry. Neurology. 2015;84(21):2185-2192. doi:10.1212/WNL.0000000000001608
5. Patrikios P, Stadelmann C, Kutzelnigg A, et al. Remyelination is extensive in a subset of multiple sclerosis patients. Brain. 2006; 129(12):3165-3172. doi:10.1093/brain/awl217
6. Skulina C, Schmidt S, Dommair K, et al. Multiple sclerosis: Brain-infiltrating CD8+ T cells persist as clonal expansions in the cerebrospinal fluid and blood. Proc Natl Acad Sci U S A. 2004;101(8):2428-2433. doi:10.1073/pnas.0308689100
7. Larochelle C, Lécuyer MA, Alvarez JI, et al. Melanoma cell adhesion molecule-positive CD8 T lymphocytes mediate central nervous system inflammation. Ann Neurol. 2015;78(1):39-53. doi:10.1002/ana.24415
8. Nave KA. Myelination and the trophic support of long axons. Nat Rev Neurosci. 2010;11(4):275-283. doi:10.1038/nrn2797
9. Siffrin V, Vogt J, Radbruch H, Nitsch R, Zipp F. Multiple sclerosis - candidate mechanisms underlying CNS atrophy. Trends Neurosci. 2010;33(4):202-210. doi:10.1016/j.tins.2010.01.002
10. G G. Multiple Sclerosis Cerebrospinal Fluid Biomarkers. Dis Markers. 2006;22(4). doi:10.1155/2006/509476
11. Fischer MT, Wimmer I, Höftberger R, et al. Disease-specific molecular events in cortical multiple sclerosis lesions. Brain. 2013;136(6):1799-1815. doi:10.1093/brain/awt110
12. Paling D, Solanky BS, Riemer F, et al. Sodium accumulation is associated with disability and a progressive course in multiple sclerosis. Brain. 2013;136(7):2305-2317. doi:10.1093/brain/awt149
13. Witte ME, Mahad DJ, Lassmann H, van Horssen J. Mitochondrial dysfunction contributes to neurodegeneration in multiple sclerosis. Trends Mol Med. 2014;20(3):179-187. doi:10.1016/j.molmed.2013.11.007
14. Dutta R, McDonough J, Yin X, et al. Mitochondrial dysfunction as a cause of axonal degeneration in multiple sclerosis patients. Ann Neurol. 2006;59(3):478-489. doi:10.1002/ana.20736
15. PRINEAS JW, KWON EE, CHO E-S, SHARER LR. Continual Breakdown and Regeneration of Myelin in Progressive Multiple Sclerosis Plaques. Ann N Y Acad Sci. 1984;436(1 Multiple Scle):11-32. doi:10.1111/j.1749-6632.1984.tb14773.x
16. Kuhlmann T, Miron V, Cuo Q, Wegner C, Antel J, Brück W. Differentiation block of oligodendroglial progenitor cells as a cause for remyelination failure in chronic multiple sclerosis. Brain. 2008;131(7):1749-1758. doi:10.1093/brain/awn096
17. Davalos D, Kyu Ryu J, Merlini M, et al. Fibrinogen-induced perivascular microglial clustering is required for the development of axonal damage in neuroinflammation. Nat Commun. 2012;3. doi:10.1038/ncomms2230
18. Piaton G, Aigrot MS, Williams A, et al. Class 3 semaphorins influence oligodendrocyte precursor recruitment and remyelination in adult central nervous system. Brain. 2011;134(4):1156-1167. doi:10.1093/brain/awr022
19. Matute C, Sánchez-Gómez MV, Martínez-Millán L, Miledi R. Glutamate receptor-mediated toxicity in optic nerve oligodendrocytes. Proc Natl Acad Sci USA. 1997;94(16):8830-8835. doi:10.1073/pnas.94.16.8830
20. Lee Y, Morrison BM, Li Y, et al. Oligodendroglia metabolically support axons and contribute to neurodegeneration. Nature. 2012;487(7408):443-448. doi:10.1038/nature11314
21. Kremer D, Küry P, Dutta R. Promoting remyelination in multiple sclerosis: Current drugs and future prospects. Mult Scler J. 2015;21(5):541-549. doi:10.1177/1352458514566419
22. Agresti C. Reversible inhibitory effects of interferon-γ and tumour necrosis factor-a on oligodendroglial lineage cell proliferation and differentiation in vitro. Eur J Neurosci. 1996;8(6):1106-1116. doi:10.1111/j. 1460-9568.1996.tb01278.x
23. Eleuteri C, Olla S, Veroni C, et al. A staged screening of registered drugs highlights remyelinating drug candidates for clinical trials. Sci Rep. 2017;7(March):1-15. doi:10.1038/srep45780
24. Mei F, Fancy SPJ, Shen Y-AA, et al. Micropillar arrays as a high-throughput screening platform for therapeutics in multiple sclerosis. Nat Med. 2014;20(8):954-960. doi:10.1038/nm.3618
25. Huang JK, Jarjour AA, Oumesmar BN, et al. Retinoid X receptor gamma signaling accelerates CNS remyelination. Nat Neurosci. 2011;14(1):45-55. doi:10.1038/nn.2702
26. Ehrlich M, Mozafari S, Glatza M, et al. Rapid and efficient generation of oligodendrocytes from human induced pluripotent stem cells using transcription factors. Proc Natl Acad Sci U S A. 2017;114(11):E2243-E2252. doi:10.1073/pnas.1614412114
27. Buchet D, Garcia C, Deboux C, Nait-Oumesmar B, Baron-Van Evercooren A. Human neural progenitors from different foetal forebrain regions remyelinate the adult mouse spinal cord. Brain. 2011;134(4):1168-1183. doi:10.1093/brain/awr030
28. Qi Y, Zhang X, Renier N, et al. Combined small-molecule inhibition accelerates the derivation of functional cortical neurons from human pluripotent stem cells. Nat Biotechnol. 2017;35(2):154-163. doi:10.1038/nbt.3777
29. Colombres M, Henríquez JP, Reig GF, et al. Heparin activates Wnt signaling for neuronal morphogenesis. J Cell Physiol. 2008;216(3):805-815. doi:10.1002/jcp.21465
30. Deshmukh VA, Tardif V, Lyssiotis CA, et al. A regenerative approach to the treatment of multiple sclerosis. Nature. 2013;502(7471):327-332. doi:10.1038/nature12647
31. Caraus I, Alsuwailem AA, Nadon R, Makarenkov V. Detecting and overcoming systematic bias in highthroughput screening technologies: A comprehensive review of practical issues and methodological solutions. Brief Bioinform. 2015;16(6):974-986. doi:10.1093/bib/bbv004
32. Telias M, Ben-Yosef D. Title: Neural differentiation is increased by GSK-3β inhibition and decreased by tankyrase inhibition in human neural precursor cells Running head: Wnt signaling in human neural precursors. doi:10.1101/509638
33. Aouadi M, Bost F, Caron L, Laurent K, Le Marchand Brustel Y, Binétruy B. p38 Mitogen-Activated Protein Kinase Activity Commits Embryonic Stem Cells to Either Neurogenesis or Cardiomyogenesis. Stem Cells. 2006;24(5):1399-1406. doi:10.1634/stemcells.2005-0398

## Claims

1. A compound able to increase oligodendrocyte precursor cell (OPC) differentiation to oligodendrocyte and/or to increase remyelination and/or to preserve neuronal viability and morphology, the compound being selected from:
a) a NK1 receptor inhibitor and/or a Sigma1 receptor inhibitor comprising: Casopitant, Aprepitant, Fosaprepitant, Rolapitant, Lanepitant and Orvepitant; and/or
b) an H3R antagonist comprising: Bavisant, Pitolisant, GSK189254, PF-03654746, A-331440, JNJ-39220675 and MK-0249; and/or
c) a CGRP antagonist comprising: Olgecepant, Telcagepant, BI 44370 TA, MK-3207, Rimegepant, SB-268262 and Ubrogepant; and/or
d) Lemborexant, PD-0325901, Vanoxerine, Indeglitazar, PAC-14028, NS-018, Rupatadine, Efatutazone Hydrochloride, Alprenolol, Danirixin, SU14813, Ezatiostat Hydrochloride, Acumapimod, Tamibarotene, Drinabant, PF-03654746, Ponesimod, Dovitinib, LY-2090314, Taladegib, Progesterone, Roxadustat, Saracatinib, Telatinib, Gandotinib, Equol, BMS-833923, Merestinib, RG7314, Adenine, Hyoscyamine, Solcitinib, Neramexane, Varlitinib, Imidafenacin, Fevipiprant, Itacitinib, Decernotinib, GSK-2636771, SSR180711, Tarenflurbil, Fluocinolone Acetonide, SB-705498, AZD1981, Raxatrigine, Octanoic Acid, Itopride, Nalfurafine hydrochloride, Istradefylline, GS-4997, AZD9056, Vatalanib, and combinations thereof;
for use in the treatment and/or prevention and/or to ameliorate symptoms of neurodegenerative diseases caused by immune-mediated demyelination.

2. The compound according to claims 1 being Casopitant and/or Bavisant and/or Telgacepant and/or Olgecepant and combinations thereof.

3. The compound according to claim 1 selected from Casopitant, Aprepitant, Fosaprepitant, Rolapitant, Lanepitant, Orvepitant and combinations thereof.

4. The compound according to claim 1 selected from Bavisant, Pitolisant, GSK189254, PF-03654746, A-331440, JNJ-39220675, MK-0249 and combinations thereof.

5. The compound according to claim 1 selected from Olgecepant, Telcagepant, BI 44370 TA, MK-3207, Rimegepant, SB-268262, Ubrogepant and combinations thereof.

6. The compound for use according to anyone of claims 1 to 5, wherein the neurodegenerative diseases caused by immune-mediated demyelination are selected from Multiple Sclerosis, Progressive Multiple sclerosis, Optic-spinal multiple sclerosis, Amyotrophic Lateral Sclerosis, Chronic relapsing inflammatory optic neuritis (CRION), Neuromyelitis optica, or Chronic inflammatory demyelinating polyneuropathy.

7. A pharmaceutical composition comprising at least one compound as defined in anyone of previous claims or combinations thereof, and a pharmaceutically acceptable carrier for use in the treatment and/or prevention of a neurodegenerative diseases caused by immune-mediated demyelination.

8. The pharmaceutical composition according to claim 7 wherein the at least one compound is at concentration of about between 100nM and 100µM.

9. The pharmaceutical composition for use according to anyone of claims 7 or 8 wherein the neurodegenerative disease caused by immune-mediated demyelination is Multiple Sclerosis, Progressive Multiple sclerosis, Optic-spinal multiple sclerosis, Amyotrophic Lateral Sclerosis, Chronic relapsing inflammatory optic neuritis (CRION), Neuromyelitis optica, or Chronic inflammatory demyelinating polyneuropathy.

10. A method for identifying a compound able to increase oligodendrocyte precursor cell (OPC) differentiation and/or to produce an expanded population of oligodendrocytes, wherein said method comprises:
- a toxicity assay on neonatal mouse oligodendrocyte progenitor cells wherein test compounds are screened for their ability to reduce [3-(4,5-dimethylthiazol-2-yl)-2,5-diphenyltetrazolium bromide (MTT)]; and/or
- a toxicity assays on rat oligodendrocyte progenitor double fluorescence CG4 line; and/or
- a differentiation assay on rat oligodendrocyte progenitor CG4 line.

11. A method for identifying a compound able to preserve neuronal viability and morphology in cell culture, wherein said method comprises:
- a toxicity assay on primary mouse cortical neurons wherein test compounds are screened in a Cell Counting Kit-8 (CCK-8) assay using WST-8 (2-(2-methoxy-4-nitrophenyl)-3-(4-nitrophenyl)-5-(2,4-disulfophenyl)-2H-tetrazolium, monosodium salt); and/or
- a neuroprotective assay on primary mouse cortical and striatal neurons wherein compounds are screened for their ability to preserve neuronal viability and morphology (neurite length and network integrity/branching) against NMDA-induced excitotoxicity; and/or
- a toxicity assay on iPSC-derived glutamatergic neurons wherein test compounds are screened in a Cell Counting Kit-8 (CCK-8) assay using WST-8 (2-(2-methoxy-4-nitrophenyl)-3-(4-nitrophenyl)-5-(2,4-disulfophenyl)-2H-tetrazolium, monosodium salt); and/or
- a neuroprotective assay on iPSC-derived glutamatergic neurons wherein compounds are screened for their ability to preserve neuronal viability against ROS (tBuOOH)-induced toxicity.

12. A method for identifying a compound able to increase neuronal differentiation and/or to produce an expanded population of neurons in cell culture, wherein said method comprises a differentiation assay on human fetal NPCs towards TUJ1+ neuronal precursors wherein:
- test compounds at 1µM concentration are co-incubated with hufNPCs for 7 days;
- 0.02% vol/vol DMSO and basal medium are used as negative controls;
- heparin is used as a positive control;
- at the end of incubation cells are fixed and analyzed via immunocytochemistry.

13. A compound identified by a method according to anyone of claims 10, 11 or 12 for use in the treatment and/or prevention and/or to ameliorate symptoms of neurodegenerative diseases caused by immune-mediated demyelination.

14. A pharmaceutical composition comprising at least one compound identified by a method according to anyone of claims 10, 11 or 12 and a pharmaceutically acceptable carrier for use in the treatment and/or prevention and/or to ameliorate symptoms of neurodegenerative diseases caused by immune-mediated demyelination.

15. The compound for use according to claim 13 or the pharmaceutical composition according to claim 14, wherein the neurodegenerative diseases caused by immune-mediated demyelination are selected from Multiple Sclerosis, Progressive Multiple sclerosis, Optic-spinal multiple sclerosis, Amyotrophic Lateral Sclerosis, Chronic relapsing inflammatory optic neuritis (CRION), Neuromyelitis optica, or Chronic inflammatory demyelinating polyneuropathy.
